(19) Europäisches Patentamt
European Patent Office
Office européen des brevets

(11) EP 3 005 940 A1

(12) EUROPEAN PATENT APPLICATION
published in accordance with Art. 153(4) EPC

(43) Date of publication:
13.04.2016 Bulletin 2016/15

(21) Application number: 14808202.7

(22) Date of filing: 17.04.2014

(51) Int Cl.:
*A61B 5/0245* (2006.01)

(86) International application number:
PCT/JP2014/002180

(87) International publication number:
WO 2014/196119 (11.12.2014 Gazette 2014/50)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME

(30) Priority: 06.06.2013 JP 2013119566
06.06.2013 JP 2013119567
06.06.2013 JP 2013119568

(71) Applicant: Seiko Epson Corporation
Shinjuku-ku
Tokyo 163-0811 (JP)

(72) Inventor: AOSHIMA, Ichiro
Suwa-shi
Nagano 392-8502 (JP)

(74) Representative: Hoffmann Eitle
Patent- und Rechtsanwälte PartmbB
Arabellastraße 30
81925 München (DE)

# (54) DEVICE FOR PROCESSING BIOLOGICAL INFORMATION, AND METHOD FOR PROCESSING BIOLOGICAL INFORMATION

(57) A pulsimeter 1 includes: a pulse rate measurement unit 110 which detects a measured pulse rate 750 about a living body of a subject; a moving rate calculation unit 120 which detects moving rate information about a movement of the subject; a reliability determination unit 160 which determines whether the reliability of the measured pulse rate 750 is high or low on the basis of a predetermined criterion; a storage unit 700 which stores a user-specific pulse estimation table 780 showing a correlation between the pulse rate and the moving rate information in association with the subject; and a table update unit 170 which updates the user-specific pulse estimation table 780. If the reliability determination unit 160 determines that the reliability of the measured pulse rate 750 is high, the table update unit 170 updates the user-specific pulse estimation table 780 on the basis of the measured pulse rate 750 and the moving rate information.

FIG. 4

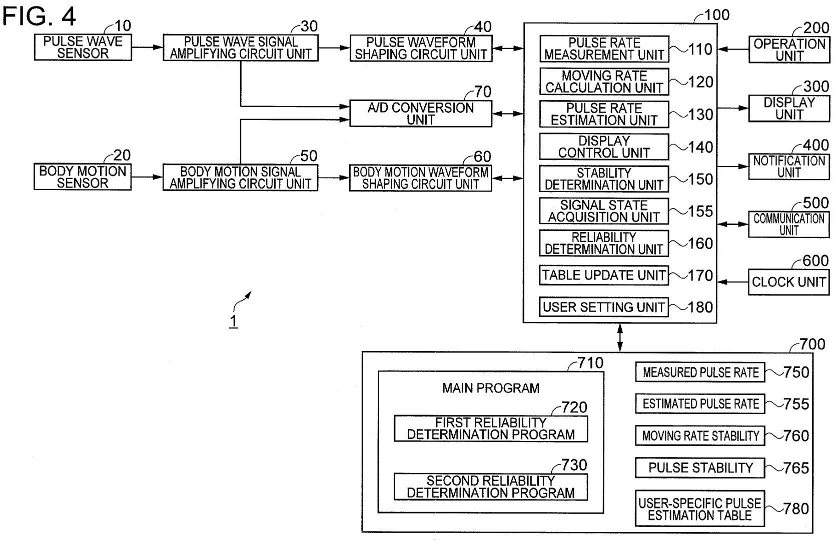


EP 3 005 940 A1

## Description

### Technical Field

The present invention relates to a biological information processing device and a biological information processing method.

### Background Art

According to the related art, as a biological information processing device used for exercise management and health management of a subject, a pulsimeter which is installed on a part of the body of the subject and measures the pulse rate of the subject is known. The pulsimeter is a device whose pulse wave sensor detects change in the amount of bloodstream of the subject wearing the device and which calculates the pulse rate of the subject and notifies the subject of the calculated pulse rate as a measurement result. As the pulse wave sensor, a sensor utilizing light, a sensor utilizing ultrasonic waves, or a sensor utilizing infrared rays or the like is known.

Since such a pulsimeter detects change in the amount of bloodstream and calculates pulse rate, the accuracy of pulse rate measurement may fall due to the physical constitution of the subject and the state of clothes or the like, in addition to irregularity in the blood flow caused by a body motion of the subject, and ambient temperature.

Also, in circumstances where the subject constantly wears the pulsimeter in everyday life, it may become difficult to measure pulse rate if the position of installation of the pulsimeter changes. That is, there can be circumstances where the position of installation of the pulsimeter comes off an ideal position of installation as a result of execution of a certain movement by the subject, making it difficult to measure pulse rate accurately.

In order to solve the problem of inability to measure pulse rate accurately, a device which detects pulse rate on the basis of a body motion of the subject in addition to detecting pulse rate with a pulse wave sensor is proposed, as disclosed in the following PTL 1. This device calculates exercise intensity from the detected body motion information and estimates pulse rate from the calculated exercise intensity on the basis of the relation between exercise intensity and pulse rate determined in advance from sample data of a number of subjects. Then, if the pulse wave sensor cannot detect pulse rate correctly, the estimated pulse rate is displayed to the subject.

### Citation List

#### Patent Literature

PTL 1: JP-A-2012-232010

### Summary of Invention

#### Technical Problem

However, the relation between exercise intensity and pulse rate determined from the sample data does not take into account differences among individuals such as athletic ability, and variation due to the use environment where the pulsimeter is used, and the like. Moreover, the relation between exercise intensity and pulse rate is predetermined and cannot be changed according to the circumstance where the pulsimeter is used. Therefore, it is difficult to improve estimation accuracy in the case of estimating pulse rate from exercise intensity.

Thus, in view of the foregoing problems, it is an object of the invention to improve the relation between exercise intensity and pulse rate according to the measurement of pulse rate, in order to achieve improved accuracy of pulse rate estimated from exercise intensity.

#### Solution to Problem

The invention is made to solve at least a part of the foregoing problems and can be realized in the following forms or application examples.

[Application Example 1]

A biological information processing device according to this application example includes: a biological information detection unit which detects biological information about a subject; a body motion information detection unit which detects body motion information about a movement of the subject; a reliability determination unit which determines reliability of the biological information on the basis of a predetermined criterion; a storage unit which stores a correlation between the body motion information and the biological information; and an update unit which updates the correlation stored in the storage unit. If the reliability determination unit determines that the biological information satisfies the predetermined criterion, the update unit updates the correlation on the basis of the biological information and the body motion information.

According to such a configuration, the correlation between biological information and body motion information is stored in the storage unit in association with the subject, and if the detected biological information is determined as satisfying a predetermined criterion, the correlation is updated on the basis of the biological information and the body motion information. Therefore, the correlation between biological information and body motion information for each subject is successively updated when biological information that satisfies the predetermined criterion is acquired. Therefore, improvement in reliability with respect to the correlation can be achieved.

[Application Example 2]

It is preferable that the biological information processing device according to the above application example includes: an estimation unit which outputs estimated biological information from the body motion information detected by the body motion information detection unit, on the basis of the correlation stored in the storage unit; a decision unit which decides one of the biological information and the estimated biological information as output information on the basis of a result of determination by the reliability determination unit; and an output unit which outputs the output information decided by the decision unit.

According to such a configuration, estimated biological information can be outputted on the basis of body motion information, and output information is decided on the basis of the reliability of biological information detected by the biological information detection unit. Therefore, highly reliable information can be outputted.

[Application Example 3]

In the biological information processing device according to the above application example, it is preferable that the reliability determination unit determines the reliability of the biological information as one of a first determination, a second determination and a third determination, on the basis of the predetermined criterion, that the decision unit decides the estimated biological information estimated by the estimation unit, as the output information, if the first determination is made on the biological information, whereas the decision unit decides the biological information detected by the biological information detection unit, as the output information, if the second determination or the third determination is made on the biological information, and that the update unit updates the correlation if the third determination is made on the biological information.

According to such a configuration, the estimated biological information estimated by the estimation unit is outputted if the reliability of the biological information detected by the biological information detection unit is determined as the first determination, whereas the biological information detected by the biological information detection unit is outputted if the reliability of the biological information is determined as the second determination or the third determination. Moreover, the correlation is updated if the reliability of the biological information is determined as the third determination. Thus, the reliability of the correlation can be improved further according to the reliability of the biological information.

[Application Example 4]

In the biological information processing device according to the above application example, the reliability determination unit may determine the reliability of the biological information on the basis of at least one of stability of the biological information detected by the biological information detection unit and noise ratio information indicating the ratio of a noise component included in a signal indicating the biological information.

[Application Example 5]

In the biological information processing device according to the above application example, it is preferable that the update unit generates a third form of the correlation on the basis of a first form of the correlation stored in the storage unit at the start of detection of the biological information by the biological information detection unit and a second form of the correlation updated from the first form of the correlation at the time of detection of the biological information by the biological information detection unit, and stores the third form of the correlation thus generated, in the storage unit.

According to such a configuration, when the detection of biological information is finished, the correlation stored in the storage unit is generated on the basis of the correlation before the detection at this time is started and the correlation updated by the detection at this time. Therefore, the influence of detection variance on the correlation can be restrained.

[Application Example 6]

In the biological information processing device according to the above application example, it is preferable that the update unit stores the third form of the correlation in the storage unit if the difference between the first form of the correlation and the second form of the correlation is within a predetermined range.

According to such a configuration, the correlation is stored if the difference between the correlation updated by the measurement at this time and the correlation up to this point is within a predetermined range. Therefore, a fall in the reliability of the correlation can be avoided.

[Application Example 7]

In the biological information processing device according to the above application example, it is preferable that the update unit does not update the correlation regardless of the result of determination by the reliability determination unit, if the relation between the biological information and the body motion information is greatly different from the correlation.

According to such a configuration, the correlation is not updated if the relation between the biological information and body motion information acquired this time departs greatly from the correlation. Therefore, a fall in the reliability of the correlation can be avoided by eliminating peculiar measurement results.

[Application Example 8]

A biological information processing method according to this application example includes: a biological information detection process of detecting biological information about a subject; a body motion information detection process of detecting body motion information about a movement of the subject; a reliability determination process of determining reliability of the biological information on the basis of a predetermined criterion; a storage process of storing a correlation between the body motion information and the biological information; and an update process of updating the correlation on the basis of the biological information and the body motion information if the biological information is determined as satisfying the predetermined criterion in the reliability determination process.

According to such a method, the correlation between biological information and body motion information is stored in the storage unit in association with the subject, and if the detected biological information is determined as satisfying a predetermined criterion, the correlation is updated on the basis of the biological information and the body motion information. Therefore, the correlation between biological information and body motion information for each subject is successively updated when biological information that satisfies the predetermined criterion is acquired. Therefore, improvement in reliability with respect to the correlation can be achieved.

[Application Example 9]

A biological information processing device according to this application example includes: a biological information detection unit which detects biological information about a subj ect; a body motion information detection unit which detects body motion information about a movement of the subject; an activity information derivation unit which derives activity information of the subject from the body motion information; a storage unit which stores a correlation between the body motion information and the biological information in association with the activity information; an estimation unit which acquires, from the storage unit, the correlation corresponding to the activity information derived by the activity information derivation unit, and decides estimated biological information from the body motion information detected by the body motion information detection unit on the basis of the correlation thus acquired; a reliability determination unit which determines reliability of the biological information; and a decision unit which decides one of the biological information and the estimated biological information as output information on the basis of a result of determination by the reliability determination unit.

According to such a configuration, the correlation between biological information and body motion information is stored in association with activity information, and estimated biological information is estimated from the body motion information on the basis of the correlation corresponding to the activity information of the subject derived from the body motion information. Then, on the basis of the reliability of the detected biological information, one of the detected biological information and the estimated biological information is decided as output information. Therefore, since the biological information decided as output information is estimated on the basis of the correlation corresponding to the activity information of the subject, improvement in reliability can be achieved.

[Application Example 10]

In the biological information processing device according to the above application example, it is preferable that the activity information derivation unit derives the activity information of the subject on the basis of periodicity of an acceleration signal acquired from the body motion information.

According to such a configuration, since the activity information of the subject is derived on the basis of the periodicity of the acceleration signal, an activity with periodicity and an activity without periodicity can be clearly discriminated from each other.

[Application Example 11]

In the biological information processing device according to the above application example, if the activity information derivation unit determines that the acceleration signal has the periodicity, the activity information derivation unit calculates an average pace during a predetermined period of time on the basis of a number of vibrations corresponding to the periodicity and determines whether the activity of the subject is walking or running, according to the value of the average pace.

According to such a configuration, in the case of an activity having periodicity, whether the activity of the subject is walking or running can be determined from the information of the average pace calculated on the basis of the number of vibrations corresponding to the periodicity.

[Application Example 12]

In the biological information processing device according to the above application example, it is preferable that if the activity information derivation unit determines that the activity of the subject is walking, the correlation corresponding to walking is acquired from the storage unit, whereas if the activity information derivation unit determines that the activity of the subject is running, the correlation corresponding to running is acquired from the storage unit.

According to such a configuration, if the activity of the subject is walking, the correlation corresponding to walking can be acquired from the storage unit, whereas if the activity of the subject is running, the correlation corresponding to running can be acquired from the storage unit.

[Application Example 13]

In the biological information processing device according to the above application example, it is preferable that if the activity information derivation unit determines that the acceleration signal does not have the periodicity, the activity information derivation unit analyzes an intensity of the acceleration signal and determines that the activity of the subject is a non-periodical exercise which causes a rise in pulse rate, on the basis of a number of occurrences of a predetermined intensity.

According to such a configuration, in the case of an activity without periodicity, by analyzing the intensity of the acceleration signal, it is possible to determine the activity of the subject as a non-periodical exercise which causes a rise in pulse rate.

[Application Example 14]

In the biological information processing device according to the above application example, it is preferable that if the activity information derivation unit determines that the activity of the subject is the non-periodical exercise which causes the rise in pulse rate, the correlation corresponding to the non-periodical exercise which causes the rise in pulse rate is acquired from the storage unit.

According to such a configuration, if the activity of the subject is the non-periodical exercise which causes the rise in pulse rate, the correlation corresponding to the non-periodical exercise which causes the rise in pulse rate can be acquired from the storage unit.

[Application Example 15]

In the biological information processing device according to the above application example, it is preferable that the decision unit decides the biological information as the output information if the reliability determination unit determines that the biological information satisfies a predetermined condition on reliability, and that the decision unit decides the estimated biological information as the output information if the reliability determination unit determines that the biological information does not satisfy the predetermined condition.

According to such a configuration, since the biological information is outputted if the reliability of the biological information is high, whereas the estimated biological information is outputted if the reliability of the biological information is low, improvement in the reliability of the output information that is outputted can be achieved.

[Application Example 16]

A biological information processing device according to this application example includes: a detection unit which detects a measured pulse rate on the basis of biological information of a subject; an estimation unit which estimates an estimated pulse rate on the basis of body motion information of the subject; a first determination unit which determines whether the measured pulse rate is included in a predetermined range prescribed on the basis of the estimated pulse rate, or not; a reliability degree information calculation unit which calculates reliability degree information on the basis of a frequency characteristic of the measured pulse rate; a second determination unit which determines whether or not the reliability degree information exceeds a predetermined reference value prescribing whether the measured pulse rate has reliability or not; and a decision unit which decides one of the measured pulse rate and the estimated pulse rate as a pulse rate of the subject on the basis of results of determination by the first determination unit and the second determination unit.

According to this application example, the first determination unit determines the measured pulse rate that is detected, on the basis of the estimated pulse rate. The estimated pulse rate is estimated on the basis of the body motion information and therefore expresses the pulse rate that changes according to the body motion information (including exercise load information). By determining whether the measured pulse rate is included in a predetermined range prescribed on the basis of the estimated pulse rate or not, it is possible to determine whether the measured pulse rate that is detected is the pulse rate that changes according to the body motion information or not. That is, if the measured pulse rate that is detected is included in the predetermined range including the estimated pulse rate, the measured pulse rate is considered to be changed according to the body motion information and therefore can be determined as an appropriate pulse rate.

The second determination unit determines whether the measured pulse rate has reliability or not, using the reliability degree information based on the frequency characteristic of the measured pulse rate. If the frequency characteristic of the measured pulse rate that is detected is exhibited intensely, the measured pulse rate is detected with a high degree of reliability and therefore can be determined as an appropriate pulse rate.

The decision unit decides one of the measured pulse rate and the estimated pulse rate as an appropriate pulse rate, considering the results of determination by both of the first determination unit and the second determination unit. The decision unit can decide the estimated pulse rate changing according to the body motion information, as an appropriate pulse rate, even if the measured pulse rate that is detected does not change according to the body motion information. That is, the pulse rate corresponding to the exercise load (body motion information) can be decided even in circumstances where the state of exercise of the subject changes.

Moreover, since the amount of information used for the determination is greater than in a determination using the result of determination by one of the first determination unit and the second determination unit, there is also an effect that the pulse rate that changes according to the body motion information can be decided with high accuracy.

[Application Example 17]

It is preferable that the first determination unit sets an upper limit value and a lower limit value on the basis of the estimated pulse rate and uses a range prescribed by the upper limit value and the lower limit value, as the predetermined range.

According to this application example, the range defined by the upper limit value and the lower limit value is a range indicating the pulse rate corresponding to the body motion information. If the measured pulse rate that is detected is within the predetermined range, the first determination unit can determine that the measured pulse rate is the pulse rate changing according to the body motion information and therefore an appropriate pulse rate.

[Application Example 18]

It is preferable that the estimation unit derives the estimated pulse rate from the body motion information, using a computational formula or correlation table that expresses a correlation between the body motion information and the estimated pulse rate.

According to this application example, the relation between the body motion information and the pulse rate changing according to the body motion information is defined by a computational formula or correlation table. Since conditions for the body motion information can be set in detail with the computational formula or correlation table, the estimated pulse rate with high accuracy can be calculated from the body motion information.

[Application Example 19]

It is preferable that the device further includes a reference value calculation unit which calculates the predetermined reference value for determining reliability of the measured pulse rate, on the basis of the result of determination by the first determination unit.
According to this application example, as the reference value for determining the reliability of the measured pulse rate is changed using the result of determination by the first determination unit, whether there is reliability or not can be determined and therefore whether the measured pulse rate is appropriate or not can be determined with high accuracy.

[Application Example 20]

It is preferable that the second determination unit determines the reliability of the measured pulse rate, using the predetermined reference value, and that the decision unit decides the measured pulse rate as the pulse rate of the subject if the measured pulse rate has reliability, and decides the estimated pulse rate as the pulse rate of the subject if the measured pulse rate does not have reliability.
According to this application example, if the measured pulse rate that is detected does not have reliability, the estimated pulse rate is an appropriate pulse rate. Even if the state of exercise of the subject changes and the reliability of the measured pulse rate that is detected is low, the estimated pulse rate which changes according to the body motion information can be decided as the pulse rate of the subject.

Brief Description of Drawings

[FIG. 1] FIG. 1 is a front view of a pulsimeter according to Embodiment 1 of the invention.
[FIG. 2] FIG. 2 (A) shows a back view of the pulsimeter. FIG. 2(B) shows a view of the state of use of the pulsimeter.
[FIG. 3] FIG. 3 is a view for explaining the operation of a pulse wave sensor.
[FIG. 4] FIG. 4 is a block diagram showing the functional configuration of the pulsimeter.
[FIG. 5] FIG. 5 is a flowchart showing the flow of main processing.
[FIG. 6] FIG. 6 is a flowchart showing the flow of first reliability determination processing.
[FIG. 7] FIG. 7 is a flowchart showing the flow of second reliability determination processing.
[FIG. 8] FIG. 8 is a block diagram showing the functional configuration of a pulsimeter according to Embodiment 2.
[FIG. 9] FIG. 9 is a view showing an example of a pulse estimation table.
[FIG. 10] FIG. 10 is a flowchart showing the flow of main processing.
[FIG. 11] FIG. 11 is a flowchart showing the flow of action analysis processing.
[FIG. 12] FIG. 12 is a flowchart showing the flow of reliability determination processing.
[FIG. 13] FIG. 13 is a graph showing the pulse rate when the state of exercise changes in Embodiment 3.
[FIG. 14] FIG. 14 is a graph showing application of window processing.
[FIG. 15] FIG. 15 is a block diagram showing an example of the functional configuration of a pulsimeter.
[FIG. 16] FIG. 16 is a flowchart showing the flow of pulse rate decision processing.
[FIG. 17] FIG. 17 is a flowchart showing the flow of pulse rate decision processing in Embodiment 4.

Description of Embodiments

Hereinafter, embodiments of the invention will be described with reference to the drawings.

(Embodiment 1)

Hereinafter, a preferred Embodiment 1 of the invention will be described with reference to the drawings. This Embodiment 1 is Embodiment 1 where a biological information processing device of the invention is applied to a wristwatch-type pulsimeter. As a matter of course, the applicable form of the invention is not limited to the embodiment described below.

1. Outer Configuration

FIG. 1 is a front view of a pulsimeter 1 according to this Embodiment 1. The pulsimeter 1 has a wristband 2, and in a case 3 thereof, a liquid crystal display unit 4 for displaying time, the operation state of the pulsimeter 1 and various kinds of information about a living body (pulse rate, exercise intensity and the like) in the form of letters, numbers, icons and the like, is arranged.

Operation buttons 5 for operating the pulsimeter 1 are arranged on circumferential parts (lateral sides) of the case 3. The pulsimeter 1 operates, for example, using a built-in secondary battery as a power source. On the lateral side of the case 3, recharging terminals 6 connected to an external charger to recharge the built-in secondary battery are arranged.

FIG. 2(A) is a back view of the pulsimeter 1, showing the appearance of the pulsimeter 1 as viewed from the back side of the case 3. FIG. 2(B) is a view of the state of use of the pulsimeter 1, showing a side view of the pulsimeter 1 in the state of being installed on a wrist WR of a subject.

On the back side of the case 3, a pulse wave sensor 10 which detects pulse waves of the subject and outputs a pulse wave signal is arranged. The pulse wave sensor 10 detects the pulse waves on a wrist WR of the subject. In this Embodiment 1, the pulse wave sensor 10 is a photoelectric pulse wave sensor and has a mechanism for optically detecting pulse waves.

FIG. 3 is a view showing the operation of the pulse wave sensor 10 and an enlarged view of the internal structure as viewed from the lateral side of the case 3. Each member in FIG. 3 is illustrated on a different scale from each other in order to show each member in a size recognizable in the illustration.

The pulse wave sensor 10 is installed in a hemispherical housing space with a circular bottom surface, formed on the back side of the case 3. Inside this housing space, a light source 12 such as an LED (light emitting diode) and a light receiving element 13 such as a phototransistor are arranged. The inner surface of the hemisphere is a mirror surface 11. If the bottom surface side of the hemisphere is defined as below, the light receiving element 13 and the light source 12 are mounted on an upper surface and a lower surface of a substrate 14, respectively.

As light Le is cast by the light source 12 toward a skin SK of the wrist WR of the user, the cast light Le is reflected by a subcutaneous blood vessel BV and returns as reflected light Lr into the hemisphere. The reflected light Lr is further reflected by the hemispherical mirror surface 11 and becomes incident on the light receiving element 13 from above.

This reflected light Lr from the blood vessel BV reflects change in the blood flow and thus changes in intensity, due to the light absorption effect of hemoglobin in the blood. The pulse wave sensor 10 switches on and off the light source 12 on a predetermined cycle that is faster than the pulsation. Then, the light receiving element 13 outputs a pulse wave signal corresponding to the intensity of received light by photoelectric conversion every time the light source 12 is switched on. The pulse wave sensor 10 switches on and off the light source 12, for example, at the frequency of 128 Hz.

Also, as shown in FIG. 2(A), the pulsimeter 1 has a built-in body motion sensor 20 for detecting body motions of the subject. In this Embodiment 1, the body motion sensor 20 is configured at least with an acceleration sensor. The acceleration sensor is a three-axis acceleration sensor taking the direction of a normal line to the cover glass surface of the case 3 as a Z-axis where the display surface side is positive, the up and down direction as a Y-axis where the direction of 12 o'clock is positive, and the left and right direction as an X-axis where the direction of 3 o' clock is positive, as shown in FIG. 1. In the state where the pulsimeter 1 is installed, the X-axis coincides with the direction from the elbow to the wrist of the subject. The body motion sensor 20 detects accelerations on the three axes of the X-axis, Y-axis and Z-axis, and outputs the results as a body motion signal.

## 2. Principles

The pulsimeter 1 measures pulse rate on the basis of the pulse wave signal detected by the pulse wave sensor 10. The pulse wave signal is a signal made up of a pulsation component signal of the subject and a body motion noise component signal superimposed on each other. Thus, the pulsimeter 1 eliminates the body motion noise component signal from the pulse wave signal and extracts the pulsation component signal, on the basis of the body motion signal outputted from the body motion sensor 20. Moreover, the pulsimeter 1 measures pulse rate on the basis of the extracted pulsation component signal.

Specifically, the pulsimeter 1 includes a digital filter such as an FIR (finite impulse response) filter, for example, as an adaptive filter, and executes processing of eliminating the body motion noise component from the pulse wave signal as digital signal processing, using the adaptive filter. The pulsimeter 1 performs frequency analysis on the extracted pulsation component signal, thus specifies a pulsation presenting spectrum, and measures pulse rate on the basis of the frequency (or period) thereof. As the frequency analysis, for example, FFT (fast Fourier transform) can be employed.

In this Embodiment 1, the circumstance where measurement of pulse rate becomes difficult when the above method is used is considered, and exercise intensity, which is one piece of the body motion information of the subject, is calculated, using the result of the measurement on the subject by the body motion sensor 20. The pulsimeter 1 estimates the pulse rate of the subject, using the calculated exercise intensity.

2-1. Estimation of Pulse Rate Using Exercise Intensity

In this Embodiment 1, the pulsimeter 1 detects acceleration of the subject as a body motion, using the acceleration sensor. The pulsimeter 1 calculates the moving rate (pace) of the subject from the acceleration detected by the acceleration sensor and thereby calculates the exercise intensity of the subject. The calculation of the moving rate can be carried out by performing predetermined frequency analysis (for example, FFT) on the acceleration signal outputted from the acceleration sensor and specifying and extracting a frequency component corresponding to the moving rate. Details of such a calculation method are disclosed, for example, in JP-A-2004-81745.

Next, the pulsimeter 1 estimates pulse rate from the moving rate on the basis of a predetermined correspondence between moving rate and pulse rate. In this Embodiment 1, an example of estimating the pulse rate corresponding to the moving rate, referring to a table showing the correlation between moving rate and pulse rate, is considered. In this table, the expected moving rate range is divided every predetermined number of moving rates (for example, several moving rates) and pulse rate corresponding to each moving rate zone is described. In this Embodiment 1, such a table (user-specific pulse estimation table 780 (FIG. 4)) is prepared for each subject. While the number of arm swings per minute is employed as the moving rate in this Embodiment 1, the number of steps per minute may also be employed.

3. Functional Configuration

FIG. 4 is a block diagram showing an example of the functional configuration of the pulsimeter 1. The pulsimeter 1 includes the pulse wave sensor 10, the body motion sensor 20, a pulse wave signal amplifying circuit unit 30, a pulse waveform shaping circuit unit 40, a body motion signal amplifying circuit unit 50, a body motion waveform shaping circuit unit 60, an A/D (analog/digital) conversion unit 70, a processing unit 100, an operation unit 200, a display unit 300, a notification unit 400, a communication unit 500, a clock unit 600, and a storage unit 700.

The pulse wave sensor 10 is a sensor which measures pulse waves of the subject with the pulsimeter 1 installed thereon, and is configured with a photoelectric pulse wave sensor, for example. The pulse wave sensor 10 detects a change in volume caused by an influx of the bloodstream into body tissues, as a pulse wave signal, and outputs the pulse wave signal to the pulse wave signal amplifying circuit unit 30.

The pulse wave signal amplifying circuit unit 30 is an amplifying circuit which amplifies the pulse wave signal inputted from the pulse wave sensor 10, with a predetermined gain. The pulse wave signal amplifying circuit unit 30 outputs the amplified pulse wave signal to the pulse waveform shaping circuit unit 40 and the A/D conversion unit 70.

The pulse waveform shaping circuit unit 40 is a circuit unit which shapes the pulse wave signal amplified by the pulse wave signal amplifying circuit unit 30 and is configured with a circuit for eliminating high-frequency noise components, a clipping circuit, and the like. The processing unit 100 determines whether pulse waves are detected or not, on the basis of the pulse waveform shaped by the pulse waveform shaping circuit unit 40.

The body motion sensor 20 is a sensor for capturing motions of the subject with the pulsimeter 1 installed thereon, and is configured at least with an acceleration sensor.

The body motion signal amplifying circuit unit 50 is an amplifying circuit which amplifies the body motion signal inputted from the body motion sensor 20, with a predetermined gain. The body motion signal amplifying circuit unit 50 outputs the amplified body motion signal to the body motion waveform shaping circuit unit 60 and the A/D conversion unit 70.

The body motion waveform shaping circuit unit 60 is a circuit unit which shapes the body motion signal amplified by the body motion signal amplifying circuit unit 50 and is configured with a circuit for eliminating high-frequency noise components, a circuit for discriminating a gravitational acceleration component from the other components, a clipping circuit, and the like. The processing unit 100 determines whether a body motion is detected or not, on the basis of the body motion waveform shaped by the body motion waveform shaping circuit unit 60.

The A/D conversion unit 70 samples and digitizes the pulse wave signal in an analog format amplified by the pulse wave signal amplifying circuit unit 30 and the body motion signal in an analog format amplified by the body motion signal amplifying circuit unit 50, each at a predetermined sampling interval, and thus converts these signals into digital signals. The A/D conversion unit 70 outputs the converted digital signals to the processing unit 100.

The processing unit 100 has the function of centrally controlling each part of the pulsimeter 1 according to various programs such as a system program stored in the storage unit 700, and has a processor such as a CPU (central processing unit). The processing unit 100 performs main processing according to a main program 710 stored in the storage unit 700 and performs control to measure or estimate the pulse rate of the subject with the pulsimeter 1 installed thereon and to cause the display unit 300 to display the pulse rate.

The processing unit 100 has a pulse rate measurement unit 110, a moving rate calculation unit 120, a pulse rate estimation unit 130, a display control unit 140, a stability determination unit 150, a signal state acquisition unit 155, a reliability determination unit 160, a table update unit 170, and a user setting unit 180, as main function units.

The pulse rate measurement unit 110 eliminates the body motion noise component from the pulse wave signal (pulse wave data) detected by the pulse wave sensor 10, using the body motion signal (body motion data) inputted from the A/D conversion unit 70, and calculates the pulse rate, which is one piece of biological information, using the extracted pulsation component (pulsation data). The calculated pulse rate is stored in the storage unit 700 as a measured pulse rate 750. The pulse rate measurement unit 110 is equivalent to the biological information detection unit which detects biological information.

The moving rate calculation unit 120 calculates the moving rate, which is one piece of the body motion information of the subject, using the body motion signal (body motion data) inputted from the A/D conversion unit 70. In this Embodiment 1, the moving rate calculation unit 120 is equivalent to the body motion information detection unit which detects body motion information about a movement of the subject. The target to be detected by the body motion information detection unit is not limited to moving rate and examples of extracting other parameters can also be considered.

The pulse rate estimation unit 130 estimates pulse rate (estimated biological information), using the moving rate calculated by the moving rate calculation unit 120. In this Embodiment 1 the pulse rate estimation unit 130 acquires the pulse rate corresponding to the moving rate calculated by the moving rate calculation unit 120, referring to the user-specific pulse estimation table 780 corresponding to the subject using the pulsimeter 1. The pulse rate thus acquired is stored in the storage unit 700 as an estimated pulse rate 755. The pulse rate estimation unit 130 is equivalent to the estimation unit.

In this Embodiment 1, the user-specific pulse estimation table 780 shows the correlation between moving rate and pulse rate in association with the subject and is stored in the storage unit 700. For example, if the moving rate of a certain subject is 98 or above and 104 or below, the pulse rate of 93 corresponds to this. If the moving rate is 105 or above and 111 or below, the pulse rate of 96 corresponds to this. Therefore, the pulse rate estimation unit 130 acquires the user-specific pulse estimation table 780 corresponding to the subject from the storage unit 700, and can acquire the pulse rate corresponding to the calculated moving rate with reference to the acquired user-specific pulse estimation table 780.

It is also possible to consider an example in which the pulse rate estimation unit 130 uses the pulse rate estimated from the moving rate, as a target pulse rate, and estimates the pulse rate in a transition state where the pulse rate gradually approaches the target pulse rate, in addition to estimating the estimated pulse rate 755 from moving rate. Here, the transition state is the state in the course of shifting from one state to another state. In this Embodiment 1, on the assumption of the situation where the subject wearing the pulsimeter 1 exercises, two patterns of state transition, that is, from the start of exercise to exercising, and from exercising to the end of exercise, can be considered as transition states.

In the transition from the start of exercise to exercising, as the subject starts exercising, the moving rate quickly rises. Accordingly, the pulse rate increases relatively fast and changes to converge to the maximum pulse rate during exercise. Meanwhile, in the transition from exercising to the end of exercise, as the subject ends exercising, the moving rate quickly falls. Accordingly, the pulse rate gently falls. Specifically, even if the exercise is ended, the pulse rate does not fall immediately and then begins to fall quickly after the lapse of a predetermined period of time. Afterward, the falling speed of the pulse rate slows down again and ultimately converges to a slightly higher value than the pulse rate at rest.

In this way, it can be understood that the change in pulse rate with time has different tendencies between the state from the start of exercise to exercising and the state from exercising to the end of exercise. Thus, by defining pulse rate estimation models in which pulse rate and moving rate shift according to the lapse of time on the assumption of each of the two cases, and selecting a model corresponding to the state, the pulse rate in the transition state can be estimated.

A detailed method for estimating the pulse rate in the transition state is disclosed, for example, in JP-A-2012-232010.

The display control unit 140 controls the display unit 300 to display the result of the measurement/estimation of pulse rate on the basis of the result of the determination by the reliability determination unit 160. That is, if the reliability determination unit 160 determines that the reliability of the result of the measurement by the pulse rate measurement unit 110 is high, the measured pulse rate 750 as output information is acquired from the storage unit 700 and displayed on the display unit 300. Meanwhile, if the reliability determination unit 160 determines that the reliability of the result of the measurement by the pulse rate measurement unit 110 is low, the estimated pulse rate 755 as output information is acquired from the storage unit 700 and displayed on the display unit 300. The display control unit 140 is equivalent to the decision unit which decides which of the measured pulse rate 750 and the estimated pulse rate 755 should be displayed.

The stability determination unit 150 determines the stability of the moving rate and pulse on the basis of the moving rate calculated by the moving rate calculation unit 120 and the pulse rate measured by the pulse rate measurement unit 110.

The stability of the moving rate is used to determine the validity of the pulse rate estimation from the moving rate. The condition for stability is, for example, that the absolute value of the difference between the moving rate measured this time and the moving rate measured previously is below a predetermined threshold. The condition for stability in terms of the stability of pulse is, for example, that the absolute value of the difference between the pulse rate measured this time and the pulse rate measured previously is below a predetermined threshold.

The result of the determination by the stability determination unit 150 is referred to by the reliability determination unit 160.

The signal state acquisition unit 155 acquires noise ratio information indicating the ratio of the detected pulse wave signal to the noise components included in the signal, on the basis of the result of the frequency analysis on the pulse wave signal. In this Embodiment 1, an example of employing an SN value indicating the ratio of noise included in the pulse wave signal (S/N) as the noise ratio information is considered. However, this is not limiting, and it is also possible to consider the case where, for example, the ratio of the pulse wave signal to noise in the case where FFT (fast Fourier transform) is performed on the pulse wave signal (MN ratio) is employed and whether reliability is high or low is determined on the basis of the continuity of the MN ratio and the variability of a predicted value. The SN value acquired by the signal state acquisition unit 155 is referred to by the reliability determination unit 160.

The SN value is defined by Pmax/N (where Pmax represents the magnitude of a baseline spectrum Pmax, and N represents the magnitude of a baseline spectrum N). As a method for detecting the SN value, for example, with respect to baseline spectra obtained by performing FFT (fast Fourier transform) on each signal, the baseline spectrum N having the tenth greatest magnitude from the maximum baseline spectrum Pmax is used as a representative of the baseline spectrum of the noise component. The reason for using this baseline spectrum N as a representative of the baseline spectrum of the noise component is that the probability of the tenth baseline spectrum being a noise is empirically high. Also, as the SN value, the value of the ratio of the magnitude of the maximum baseline spectrum Pmax to the magnitude of the baseline spectrum N is employed.

In the above example, the baseline spectrum N having the tenth greatest magnitude from the maximum baseline spectrum Pmax is used as a representative of the baseline spectrum of the noise component. However, the invention is not limited to this, and another baseline spectrum, for example, the seventh baseline spectrum may be used as a representative of the noise component.

The reliability determination unit 160 determines whether the measured pulse rate 750 has reliability or not, on the basis of the stability of the moving rate and the stability of the pulse determined by the stability determination unit 150 and the information acquired by the signal state acquisition unit 155, as predetermined criteria for determination. However, the information for determining reliability is not limited to these.

Also, in this Embodiment 1, the reliability determination unit 160 performs reliability determination processing in two stages on the measured pulse rate 750 and thereby determines one of a first determination ("low"), a second determination ("high") and a third determination ("extremely high"), in order from the lowest reliability.

That is, in the first reliability determination processing, whether the reliability of the measured pulse rate 750 is "high" or "low" is determined on the basis of the determination on whether the measured pulse rate 750 is within an expected range or not and the determination on whether the SN value acquired by the signal state acquisition unit 155 is above a predetermined criterion (first criterion) or not. That is, whether the measured pulse rate 750 satisfies a predetermined condition for reliability or not is determined. In this Embodiment 1, the pulse rate to be displayed on the display unit 300 as the pulse rate of the subject is decided on the basis of the result of the determination in this first reliability determination processing.

Meanwhile, in the second reliability determination processing, with respect to the measured pulse rate 750 whose reliability is determined as "high" in the first reliability determination processing, whether the reliability of the measured pulse rate 750 is "extremely high" or not is determined on the basis of the determination on whether the SN value is above a stricter criterion (second criterion) than the first criterion or not and the determination on whether the pulse rate and moving rate are stable or not. The result of the determination is sent to the table update unit 170.

In this Embodiment 1, "0.5" is employed as the first criterion, of the criteria for determination on the SN value, and "2.0" is employed as the second criterion. However, these values are not limiting.

Moreover, the respective determinations in the first reliability determination processing and the second reliability determination processing are not limited to the combination described above, and various examples including the determination on the MN ratio can be considered as combinations of determinations.

The table update unit 170 updates the user-specific pulse estimation table 780 on the basis of the result of the reliability determination by the reliability determination unit 160. The table update unit 170 is equivalent to the update unit.

In this Embodiment 1, if the reliability determination unit 160 determines that the reliability of the measured pulse rate 750 is "extremely high", the relation between the moving rate of the subject calculated by the moving rate calculation unit 120 and the measured pulse rate 750 is added to the user-specific pulse estimation table 780. As a result, the user-specific pulse estimation table 780 successively learns the relation between the moving rate and the measured pulse rate 750 whose reliability is determined as "extremely high".

In this way, as the subject continues using the pulsimeter 1, the user-specific pulse estimation table 780 is updated and the relation between the moving rate and the measured pulse rate 750 with extremely high reliability is reflected on the user-specific pulse estimation table 780. Therefore, as the pulse rate of the subject is measured, the reliability of the user-specific pulse estimation table 780 is gradually improved and the estimation accuracy of the pulse rate estimation unit 130 is gradually improved as well.

Also, the table update unit 170 may further include the following functions. That is, the table update unit 170 acquires, from the storage unit 700, the user-specific pulse estimation table 780 before the start of measurement of pulse rate after the pulsimeter 1 is started up, and holds the user-specific pulse estimation table 780 in a predetermined place. Then, if a pulse rate with high reliability is acquired, the updated user-specific pulse estimation table 780 is compared with the user-specific pulse estimation table 780 held in the predetermined place, and the user-specific pulse estimation table 780 is updated further on the basis of the result of the comparison. The updated user-specific pulse estimation table 780 is stored in the storage unit 700.

For example, average values of table values in the user-specific pulse estimation table 780 before the start of measurement and the user-specific pulse estimation table 780 at the end of measurement may be calculated respectively, and the user-specific pulse estimation table 780 may be updated, using the calculated average values as the respective table values. Here, if the difference in the respective table values is above a predetermined reference, a configuration in which the range of change in the table values is compressed to approximately 25% of the difference or a configuration in which the user-specific pulse estimation table 780 is not updated can be considered.

Also, in the case where pulse rates are measured more than a predetermined number of times, or where the user-specific pulse estimation table 780 is updated more than a predetermined number of times, if the moving rate and the measured pulse rate 750 to be added to the user-specific pulse estimation table 780 largely deviate from the table values in the existing user-specific pulse estimation table 780, a configuration in which the table update unit 170 does not update the user-specific pulse estimation table 780 can be considered. Consequently, if the user uses the device in a special environment such as on a high mountain, error factors in the pulse estimation processing can be eliminated by not incorporating but excluding the acquired data from the user-specific pulse estimation table 780. Thus, the reliability of the user-specific pulse estimation table 780 can be improved further.

The user setting unit 180 carries out settings on the subject wearing the pulsimeter 1. In this Embodiment 1, at the time of initial setting or in the case where the subject wearing the pulsimeter 1 is changed, the user operates the operation unit 200 and carries out the setting. In this case, for example, the user inputs information that enables identification of him/herself, via the operation unit 200, and if the user-specific pulse estimation table 780 corresponding to the inputted information is stored in the storage unit 700, the user is recognized as an existing user, and the user-specific pulse estimation table 780 corresponding to this user is referred to when the pulse rate estimation unit 130 estimates pulse rate.

Meanwhile, if the user-specific pulse estimation table 780 corresponding to the user who carries out the setting is not stored in the storage unit 700, a user-specific pulse estimation table 780 corresponding to the user is newly prepared. The newly prepared user-specific pulse estimation table 780 may be a data table showing the relation between moving rate and pulse rate decided on the basis of sample data gathered from a large number of subjects. Also, at the time of user setting, the subject may be instructed to perform a predetermined movement (for example, exercise at a predetermined moving rate on flatland), and the user-specific pulse estimation table 780 may be prepared on the basis of the result of the predetermined movement by the subject.

The operation unit 200 is an input device configured with button switches or the like and outputs the signal of a button that is pressed, to the processing unit 100. With operations on this operation unit 200, various instructions such as a pulse measurement instruction are inputted. The operation unit 200 is equivalent to the operation buttons 5 in FIG. 1.

The display unit 300 is a display device which is configured with an LCD (liquid crystal display) or the like, as one output unit, and which performs various displays based on display signals inputted from the processing unit 100. Various kinds of information about a living body (pulse rate, exercise intensity and the like) are displayed on the display unit 300. The display unit 300 is equivalent to the liquid crystal display unit 4 in FIG. 1.

The notification unit 400 is a notification device which is configured with a speaker, a piezoelectric oscillator and the like and which issues various notifications based on notification signals inputted from the processing unit 100. For example, the notification unit 400 outputs an alarm sound from the speaker or causes the piezoelectric oscillator to oscillate, thus issuing various notifications to the subject.

The communication unit 500 is a communication device for transmitting and receiving information utilized inside the device, to and from an external information processing device such as a personal computer (PC), under the control of the processing unit 100. As the communication method of this communication unit 500, various methods can be applied, such as a method of wired connection via a cable conforming to a predetermined communication standard, a method of connection via an intermediate device which also serves as a charger, called a cradle, and a method of wireless connection using near field radio communication.

Also, a configuration in which various kinds of information about a living body are outputted in the form of sounds, or transmitted to an information processing device, using the notification unit 400 and the communication unit 500 as output units, can also be considered.

The clock unit 600 is a clock device which is configured with a crystal oscillating device made up of a crystal oscillator and an oscillation circuit, and which measures time of the pulsimeter 1. The time measured by the clock unit 600 is constantly outputted to the processing unit 100.

The storage unit 700 is configured by a storage device such as a ROM (read only memory), flash ROM, or RAM (random access memory), and stores the system program of the pulsimeter 1 and various programs and data or the like for realizing various functions such as the pulse rate measurement/estimation function and the exercise intensity measurement function. Also, the storage unit 700 has a work area for temporarily storing data currently being processed in various kinds of processing, and the results of processing or the like.

4. Functional Configuration

As shown in FIG. 4, the main program 710 executed as main processing (FIG. 5) is stored in the storage unit 700. The main program 710 includes, as sub-routines, a first reliability determination program 720 and a second reliability determination program 730 for determining the reliability of the measured pulse rate 750.

Also, the measured pulse rate 750, the estimated pulse rate 755, moving rate stability 760, pulse stability 765, and the user-specific pulse estimation table 780 are stored as data in the storage unit 700.

5. Flow of Processing

FIG. 5 is a flowchart showing the flow of the main processing (biological information processing method) executed in the pulsimeter 1 by the processing unit 100 reading out the main program 710 stored in the storage unit 700.

First, the processing unit 100 carries out initial setting (Step S800). Specifically, various calculation parameters used in the main processing (for example, moving rate stability 760 and pulse stability 765), and the values of the measured pulse rate 750 and the estimated pulse rate 755 stored in the storage unit 700 are initialized.

Next, the processing unit 100 decides the user-specific pulse estimation table 780 to be referred to at the time of pulse estimation, corresponding to the user using this pulsimeter 1, that is, the subject set by the user setting unit 180 (Step S802).

Then, the processing unit 100 acquires the results of detection by the pulse wave sensor 10 and the body motion sensor 20 (Step S804).

Next, the processing unit 100 starts moving rate calculation processing in which the moving rate of the subject is calculated, using the result of the detection by the body motion sensor 20 (Step S806) <body motion information detection process>.

Next, the processing unit 100 estimates the pulse rate from the moving rate of the subject calculated by the moving rate calculation unit 120, referring to the user-specific pulse estimation table 780 corresponding to the subject (Step S808), and stores the pulse rate that is estimated in the storage unit 700 as the estimated pulse rate 755.

Then, the processing unit 100 starts pulse rate measurement processing in which the pulse rate of the subject is measured, using the result of the detection of the pulse wave signal by the pulse wave sensor 10 and the result of the detection by the body motion sensor 20, and then calculates the pulse rate from the result of the measurement and stores the calculated pulse rate in the storage unit 700 as the measured pulse rate 750 (Step S810) <biological information detection process>.

Next, the processing unit 100 carries out reliability determination processing on the measured pulse rate 750 according to the first reliability determination program 720 stored in the storage unit 700 (step S850).

FIG. 6 is a flowchart showing the flow of the first reliability determination processing.

First, the reliability determination unit 160 determines whether the measured pulse rate 750 is within a predetermined range or not (Step S852). The predetermined range is a preset range indicating an expected range of pulse rates. Here, if it is determined that the measured pulse rate 750 is within the predetermined range (Yes in Step S852), the reliability determination unit 160 acquires the SN value of the pulse wave signal (Step S854).

The reliability determination unit 160 determines whether the SN value satisfies a predetermined first criterion or not (Step S856). If the SN value is determined as satisfying the predetermined first criterion (Yes in Step S856), the reliability determination unit 160 determines that the reliability of the measured pulse rate 750 is "high" (Step S858) and ends the first reliability determination processing.

Meanwhile, if it is determined in Step S852 that the measured pulse rate 750 is not within the predetermined rage (No), or if it is determined in Step S856 that the SN value does not satisfy the predetermined first criterion (No), the reliability determination unit 160 determines that the reliability of the measured pulse rate 750 is "low" (Step S860) and ends the first reliability determination processing.

Back to FIG. 5, the processing unit 100 determines whether the reliability determination unit 160 evaluates the reliability of the measured pulse rate 750 as "high" or not (Step S812).

Here, if the reliability of the measured pulse rate 750 is evaluated as "low" (No in Step S812), the processing unit 100 acquires the estimated pulse rate 755 from the storage unit 700 as the pulse rate of the subject, then displays the estimated pulse rate 755 on the display unit 300 (Step S820), and goes to Step S822.

Meanwhile, if the reliability of the measured pulse rate 750 is evaluated as "high" (Yes in Step S812), the processing unit 100 acquires the measured pulse rate 750 from the storage unit 700 as the pulse rate of the subject and displays the measured pulse rate 750 on the display unit 300 (Step S814).

Next, the processing unit 100 carries out reliability determination processing on the measured pulse rate 750 according to the second reliability determination program 730 stored in the storage unit 700 (Step S880).

FIG. 7 is a flowchart showing the flow of the second reliability determination processing (reliability determination process).

First, the reliability determination unit 160 determines whether the SN value satisfies a second criterion that is stricter than the first criterion, or not (Step S882).

Here, if the SN value is determined as satisfying the second criterion (Yes in Step S882), the reliability determination unit 160 acquires the pulse stability 765 determined by the stability determination unit 150, from the storage unit 700, and determines the stability of the measured pulse rate 750 (Step S884).

Here, if the measured pulse rate 750 is determined as stable (Yes in Step 886), the reliability determination unit 160 acquires the moving rate stability 760 determined by the stability determination unit 150, from the storage unit 700, and determines the stability of the moving rate (Step S888).

Here, if the moving rate is determined as stable (Yes in Step S888), the reliability determination unit 160 determines that the reliability of the measured pulse rate 750 is "extremely high" (Step S892) and ends the second reliability determination processing.

Meanwhile, if it is determined in Step S882 that the SN value does not satisfy the second criterion (No), or if it is determined in Step S886 that the measured pulse rate 750 is not stable (No), or if it is determined in Step S890 that the moving rate is not stable (No), the reliability determination unit 160 ends the processing without determining that the reliability of the measured pulse rate 750 is "extremely high". In this case, the result of the determination that the reliability of the measured pulse rate 750 is "high" is maintained.

Back to FIG. 5, the processing unit 100 determines whether the reliability determination unit 160 evaluates the reliability of the measured pulse rate 750 as "extremely high" or not (Step S816).

Here, if the reliability determination unit 160 evaluates the reliability of the measured pulse rate 750 as "extremely high" (Yes in Step 816), the user-specific pulse estimation table 780 is updated on the basis of the relation between the moving rate and the measured pulse rate 750 (Step S818) <update process>, and processing goes to Step S822.

Meanwhile, if the reliability determination unit 160 does not evaluate the reliability of the measured pulse rate 750 as "extremely high" (No in Step S816), the processing goes to Step S822.

In Step S822, the processing unit 100 determines whether to end the main processing or not. If the main processing is not to end (No in Step S822), the processing returns to Step S804.

Meanwhile, if the main processing is to end (Yes in Step S822), the processing unit 100 sets the user-specific pulse estimation table 780 and an initial value to be used next time the main processing is executed (Step S824) , and the processing unit 100 ends the main processing.

The above-described Embodiment 1 has the following effects.

(1) The user-specific pulse estimation table 780 used to estimate pulse rate is prepared for each subject and updated according to the use. Therefore, the user-specific pulse estimation table 780 acquires features taking the exercise ability and state of use varying from subject to subject into account, as the subject uses the pulsimeter 1. Consequently, the estimation accuracy of the pulse rate of each subject estimated from the user-specific pulse estimation table 780 is improved as the subject uses the pulsimeter 1.

(2) The reliability determination unit 160 determines the reliability of the measured pulse rate 750 calculated on the basis of the pulse wave signal detected by the pulse wave sensor 10. If the measured pulse rate 750 is determined as extremely reliable as a result of the determination, the table update unit 170 updates the user-specific pulse estimation table 780 on the basis of the relation between the moving rate and the measured pulse rate 750 of the subject. Therefore, since the user-specific pulse estimation table 780 is updated only if the measured pulse rate 750 is reliable, the reliability of table values is improved as the user-specific pulse estimation table 780 is updated, and therefore improvement in the estimation accuracy of pulse rate can be achieved.

(Embodiment 2)

Next, Embodiment 2 of the invention will be described with reference to FIGS. 8 to 12. In the description below, the same parts as the parts that are already described are denoted by the same reference numbers and the description thereof is omitted.

1. Principles

A pulsimeter 1 to which the biological information processing device of the invention is applied measures pulse rate on the basis of the pulse wave signal detected by a pulse wave sensor 10. The pulse wave signal is a signal made up of a pulsation component signal of the subject and a body motion noise component signal superimposed on each other. Thus, the pulsimeter 1 eliminates the body motion noise component signal from the pulse wave signal and extracts the pulsation component signal, on the basis of the body motion signal outputted from a body motion sensor 20. The pulsimeter 1 measures pulse rate on the basis of the extracted pulsation component signal.

Specifically, the pulsimeter 1 includes a digital filter such as an FIR (finite impulse response) filter, for example, as an adaptive filter, and executes processing of eliminating the body motion noise component from the pulse wave signal as digital signal processing, using the adaptive filter. The pulsimeter 1 performs frequency analysis on the extracted pulsation component signal, thus specifies a pulsation presenting spectrum, and measures pulse rate on the basis of the frequency (or period) thereof. As the frequency analysis, for example, FFT (fast Fourier transform) can be employed.

In this Embodiment 2, the circumstance where measurement of pulse rate becomes difficult when the above method is used is considered, and exercise intensity, which is one piece of the body motion information of the subject, is calculated, using the result of the measurement on the subject by the body motion sensor 20. The pulsimeter 1 estimates the pulse rate of the subject, using the calculated exercise intensity.

1-1. Estimation of Pulse Rate Using Exercise Intensity

In this Embodiment 2, the pulsimeter 1 detects acceleration of the subject as a body motion, using an acceleration sensor. The pulsimeter 1 calculates the pace (moving rate) of the subject from the acceleration detected by the acceleration sensor and thereby calculates the exercise intensity of the subject. The calculation of the moving rate can be carried out by performing predetermined frequency analysis (for example, FFT) on the acceleration signal outputted from the acceleration sensor and specifying and extracting a frequency component corresponding to the moving rate. Details of such a calculation method are disclosed, for example, in JP-A-2004-81745.

Next, the pulsimeter 1 estimates pulse rate from the moving rate on the basis of a predetermined correspondence between moving rate and pulse rate. In this Embodiment 2, an example of estimating the pulse rate corresponding to the moving rate, referring to a table showing the correlation between moving rate and pulse rate, is considered.

1-2. Discrimination of Action Based on Body Motion Signal

Acceleration data on the three axes of the X-axis, Y-axis and Z-axis obtained from the acceleration sensor provided in the body motion sensor 20 include change in the motion of the subject. By properly selecting the axis to be referred to, it is possible to discriminate between similar actions. Therefore, the pulsimeter 1 decides, as a main axis, an axis which includes the greatest change in motion during every predetermined period from among the three axes, then analyzes the acceleration data on the main axis and the calculated moving rate, and thus can discriminate an action pattern of the subject.

1-3. Association between Action and Pulse Rate

Various action patterns of the subject can be considered and the intensity of exercise varies from one action pattern to another. Therefore, the pulsimeter 1 can estimate pulse rate accurately by prescribing the correlation between moving rate and pulse rate for each action pattern.

2. Functional Configuration

FIG. 8 is a block diagram showing an example of the functional configuration of the pulsimeter 1. The pulsimeter 1 includes the pulse wave sensor 10, the body motion sensor 20, a pulse wave signal amplifying circuit unit 30, a pulse waveform shaping circuit unit 40, a body motion signal amplifying circuit unit 50, a body motion waveform shaping circuit unit 60, an A/D (analog/digital) conversion unit 70, a processing unit 100, an operation unit 200, a display unit 300, a notification unit 400, a communication unit 500, a clock unit 600, and a storage unit 700.

The pulse wave sensor 10 is a sensor which measures pulse waves of the subject with the pulsimeter 1 installed thereon, and is configured with a photoelectric pulse wave sensor, for example. The pulse wave sensor 10 detects a change in volume caused by an influx of the bloodstream into body tissues, as a pulse wave signal, and outputs the pulse wave signal to the pulse wave signal amplifying circuit unit 30.

The pulse wave signal amplifying circuit unit 30 is an amplifying circuit which amplifies the pulse wave signal inputted from the pulse wave sensor 10, with a predetermined gain. The pulse wave signal amplifying circuit unit 30 outputs the amplified pulse wave signal to the pulse waveform shaping circuit unit 40 and the A/D conversion unit 70.

The pulse waveform shaping circuit unit 40 is a circuit unit which shapes the pulse wave signal amplified by the pulse wave signal amplifying circuit unit 30 and is configured with a circuit for eliminating high-frequency noise components, a clipping circuit, and the like. The processing unit 100 determines whether pulse waves are detected or not, on the basis of the pulse waveform shaped by the pulse waveform shaping circuit unit 40.

The body motion sensor 20 is a sensor for capturing motions of the subject with the pulsimeter 1 installed thereon. In this Embodiment 2, the body motion sensor 20 has an acceleration sensor for detecting acceleration in each of the X-axis direction, Y-axis direction and Z-axis direction.

The body motion signal amplifying circuit unit 50 is an amplifying circuit which amplifies the body motion signal inputted from the body motion sensor 20, with a predetermined gain. The body motion signal amplifying circuit unit 50 outputs the amplified body motion signal to the body motion waveform shaping circuit unit 60 and the A/D conversion unit 70.

The body motion waveform shaping circuit unit 60 is a circuit unit which shapes the body motion signal amplified by the body motion signal amplifying circuit unit 50 and is configured with a circuit for eliminating high-frequency noise components, a circuit for discriminating a gravitational acceleration component from the other components, a clipping circuit, and the like. The processing unit 100 determines whether a body motion is detected or not, on the basis of the body motion waveform shaped by the body motion waveform shaping circuit unit 60.

The A/D conversion unit 70 samples and digitizes the pulse wave signal in an analog format amplified by the pulse wave signal amplifying circuit unit 30 and the body motion signal in an analog format amplified by the body motion signal amplifying circuit unit 50, each at a predetermined sampling interval, and thus converts these signals into digital signals. The A/D conversion unit 70 outputs the converted digital signals to the processing unit 100.

The processing unit 100 has the function of centrally controlling each part of the pulsimeter 1 according to various programs such as a system program stored in the storage unit 700, and has a processor such as a CPU (central processing unit). The processing unit 100 performs main processing according to a main program 710 stored in the storage unit 700 and performs control to measure or estimate the pulse rate of the subject with the pulsimeter 1 installed thereon and to cause the display unit 300 to display the pulse rate.

The processing unit 100 has a pulse rate measurement unit 110, an action analysis unit 115, a moving rate calculation unit 120, a pulse rate estimation unit 130, a display control unit 140, a signal state acquisition unit 155, and a reliability determination unit 160, as main function units.

The pulse rate measurement unit 110 eliminates the body motion noise component from the pulse wave signal (pulse wave data) detected by the pulse wave sensor 10, using the body motion signal (body motion data) inputted from the A/D conversion unit 70, and calculates the pulse rate, which is one piece of biological information, using the extracted pulsation component (pulsation data). The calculated pulse rate is stored in the storage unit 700 as a measured pulse rate 750. The pulse rate measurement unit 110 is equivalent to the biological information detection unit which detects pulse rate as biological information.

The action analysis unit 115 analyzes the body motion signal inputted from the A/D conversion unit 70, thus extracts acceleration data indicating the acceleration of the body motion from the body motion signal, and analyzes the action (activity information) of the subject on the basis of the extracted acceleration data and the moving rate calculated by the moving rate calculation unit 120. In this Embodiment 2, the action analysis unit 115 determines one of "walking", "running", "calisthenics" and "other" as the action of the subject on the basis of the result of the analysis. Here, the "calisthenics" refers to a non-periodical exercise which causes a rise in pulse rate, and the "other" refers to a movement which does not cause a rise in pulse rate. The action analysis unit 115 is equivalent to the activity information derivation unit.

The moving rate calculation unit 120 calculates the moving rate, which is one piece of the body motion information of the subject, using the body motion signal (body motion data) inputted from the A/D conversion unit 70. The moving rate calculation unit 120 is equivalent to the body motion information detection unit which detects body motion information about a movement of the subject. The target to be detected by the body motion information detection unit is not limited to moving rate and examples of extracting other parameters can also be considered.

The pulse rate estimation unit 130 estimates pulse rate (estimated biological information), using the moving rate calculated by the moving rate calculation unit 120. In this Embodiment 2, the pulse rate estimation unit 130 acquires the pulse rate corresponding to the moving rate calculated by the moving rate calculation unit 120, referring to an action-specific pulse estimation table 780 corresponding to the action of the subject. The pulse rate thus acquired is stored in the storage unit 700 as an estimated pulse rate 755. The pulse rate estimation unit 130 is equivalent to the estimation unit.

In this Embodiment 2, as the action-specific pulse estimation table 780, numeric data for estimating pulse rate from moving rate is stored in association with the action of the subject, in the storage unit 700. The pulse rate estimation unit 130 acquires the action-specific pulse estimation table 780 corresponding to the action of the subject from the storage unit 700. FIG. 9 shows an example of the pulse estimation tables for walking, running, and calisthenics.

In the cases of walking and running, the relation between moving rate and pulse rate is described in the action-specific pulse estimation table 780. Also, as an average moving rate during a predetermined time of approximately one minute, the moving rate of 90 times per minute or above, and below 140 times per minute, is assumed in the case of walking, and the moving rate of 140 times per minute or above is assumed in the case of running. The moving rate range in the case of walking is 6 times per minute and the moving rate range in the case of running is 3 times per minute. Thus, the moving rate range in the case of walking is set to be greater than the moving rate range in the case of running.

Meanwhile, in the case of calisthenics, the relation between intensity of acceleration obtained by successively accumulating differences in acceleration data calculated from the body motion signal corresponding to ten-plus seconds, and pulse rate, is described in the action-specific pulse estimation table 780. In this Embodiment 2, a range of intensity of acceleration of 800 or above and 24000 or below is divided into eight ranges, and the pulse rate corresponding to each range is described.

Back to FIG. 8, the pulse rate estimation unit 130 acquires the pulse rate corresponding to the moving rate or intensity of acceleration in question, referring to the action-specific pulse estimation table 780 corresponding to the action determined by the action analysis unit 115, and defines the acquired pulse rate as the estimated pulse rate 755.

It is also possible to consider an example in which the pulse rate estimation unit 130 uses the pulse rate estimated from the moving rate, as a target pulse rate, no matter which action is determined by the action analysis unit 115, and estimates the pulse rate in a transition state where the pulse rate gradually approaches the target pulse rate with the lapse of time. The transition state is the state in the course of shifting from one state to another state. In this Embodiment 2, on the assumption of the situation where the subject wearing the pulsimeter 1 exercises, two patterns of state transition, that is, from the start of exercise to exercising, and from exercising to the end of exercise, can be considered as transition states.

In the transition from the start of exercise to exercising, as the subject starts exercising, the moving rate quickly rises. Accordingly, the pulse rate increases relatively fast and changes to converge to the maximum pulse rate during exercise. Meanwhile, in the transition from exercising to the end of exercise, as the subject ends exercising, the moving rate quickly falls. Accordingly, the pulse rate gently falls. Specifically, even if the exercise is ended, the pulse rate does not fall immediately and then begins to fall quickly after the lapse of a predetermined period of time. Afterward, the falling speed of the pulse rate slows down again and ultimately converges to a slightly higher value than the pulse rate at rest.

In this way, it can be understood that the change in pulse rate with time has different tendencies between the state from the start of exercise to exercising and the state from exercising to the end of exercise. Thus, by defining pulse rate estimation models in which pulse rate and moving rate shift according to the lapse of time on the assumption of each of the two cases, and selecting a model corresponding to the state, the pulse rate in the transition state can be estimated.

A detailed method for estimating the pulse rate in the transition state is disclosed, for example, in JP-A-2012-232010.

The display control unit 140 controls the display unit 300 to display the result of the measurement/estimation of pulse rate on the basis of the result of the determination by the reliability determination unit 160. That is, if the reliability determination unit 160 determines that the reliability of the result of the measurement by the pulse rate measurement unit 110 is high, the measured pulse rate 750 as output information is acquired from the storage unit 700 and displayed on the display unit 300 as the pulse rate of the subject. Meanwhile, if the reliability determination unit 160 determines that the reliability of the result of the measurement by the pulse rate measurement unit 110 is low, the estimated pulse rate 755 as output information is acquired from the storage unit 700 and displayed on the display unit 300 as the pulse rate of the subject. The display control unit 140 is equivalent to the decision unit which decides which of the measured pulse rate 750 and the estimated pulse rate 755 should be displayed.

The signal state acquisition unit 155 acquires noise content information indicating the content relation between the detected pulse wave signal and the noise components included in the signal, on the basis of the result of the frequency analysis on the pulse wave signal. In this Embodiment 2, an example of employing an SN value 770 indicating the ratio of noise included in the pulse wave signal (S/N) as the noise content information is considered. However, this is not limiting, and it is also possible to consider the case where, for example, the ratio of the pulse wave signal to noise in the case where FFT (fast Fourier transform) is performed on the pulse wave signal (MN ratio) is employed and whether reliability is high or low is determined on the basis of the continuity of the MN ratio and the variability of a predicted value. The SN value 770 acquired by the signal state acquisition unit 155 is stored in the storage unit 700 and referred to by the reliability determination unit 160.

The SN value is defined by Pmax/N (where Pmax represents the magnitude of a baseline spectrum Pmax, and N represents the magnitude of a baseline spectrum N). As a method for detecting the SN value 770, for example, with respect to baseline spectra obtained by performing FFT (fast Fourier transform) on each signal, the baseline spectrum N having the tenth greatest magnitude from the maximum baseline spectrum Pmax is used as a representative of the baseline spectrum of the noise component. The reason for using this baseline spectrum N having the tenth greatest magnitude as a representative of the baseline spectrum of the noise component is that the probability of the tenth baseline spectrum being a noise is empirically high. Also, as the SN value 770, the value of the ratio of the magnitude of the maximum baseline spectrum Pmax to the magnitude of the baseline spectrum N is employed.

In the above example, the baseline spectrum N having the tenth greatest magnitude from the maximum baseline spectrum Pmax is used as a representative of the baseline spectrum of the noise component. However, the invention is not limited to this, and another baseline spectrum, for example, the seventh baseline spectrum may be used as a representative of the noise component.

The reliability determination unit 160 determines whether the reliability of the measured pulse rate 750 is high or low, on the basis of the SN value 770 acquired by the signal state acquisition unit 155, as a predetermined criterion. That is, the reliability determination unit 160 determines whether the reliability of the measured pulse rate 750 is "high" or "low", on the basis of the determination on whether the measured pulse rate 750 is within an expected range or not and the determination on whether the SN value 770 acquired by the signal state acquisition unit 155 is above a predetermined criterion or not. That is, whether the measured pulse rate 750 satisfies a predetermined condition for reliability or not is determined. In this Embodiment 2, the pulse rate to be displayed on the display unit 300 as the pulse rate of the subject is decided on the basis of the result of this determination.

In this Embodiment 2, "0.5" is employed as the predetermined criterion for determination on the SN value 770. However, this value is not limiting.

Moreover, the determination by the reliability determination unit 160 is not limited to the combination described above, and combinations of various determinations including determination on the MN ratio can be considered.

The operation unit 200 is an input device configured with button switches or the like and outputs the signal of a button that is pressed, to the processing unit 100. With operations on this operation unit 200, various instructions such as a pulse measurement instruction are inputted. The operation unit 200 is equivalent to the operation buttons 5 in FIG. 1.

The display unit 300 is a display device which is configured with an LCD (liquid crystal display) or the like, as one output unit, and which performs various displays based on display signals inputted from the processing unit 100. Various kinds of information about a living body (pulse rate, exercise intensity and the like) are displayed on the display unit 300. The display unit 300 is equivalent to the liquid crystal display unit 4 in FIG. 1.

The notification unit 400 is a notification device which is configured with a speaker, a piezoelectric oscillator and the like and which issues various notifications based on notification signals inputted from the processing unit 100. For example, the notification unit 400 outputs an alarm sound from the speaker or causes the piezoelectric oscillator to oscillate, thus issuing various notifications to the subject.

The communication unit 500 is a communication device for transmitting and receiving information utilized inside the device, to and from an external information processing device such as a personal computer (PC), under the control of the processing unit 100. As the communication method of this communication unit 500, various methods can be applied, such as a method of wired connection via a cable conforming to a predetermined communication standard, a method of connection via an intermediate device which also serves as a charger, called a cradle, and a method of wireless connection using near field radio communication.

Also, a configuration in which various kinds of information about a living body are outputted in the form of sounds, or transmitted to an information processing device, using the notification unit 400 and the communication unit 500 as output units, can also be considered.

The clock unit 600 is a clock device which is configured with a crystal oscillating device made up of a crystal oscillator and an oscillation circuit, and which measures time of the pulsimeter 1. The time measured by the clock unit 600 is constantly outputted to the processing unit 100.

The storage unit 700 is configured by a storage device such as a ROM (read only memory), flash ROM, or RAM (random access memory), and stores the system program of the pulsimeter 1 and various programs and data or the like for realizing various functions such as the pulse rate measurement/estimation function and the exercise intensity measurement function. Also, the storage unit 700 has a work area for temporarily storing data currently being processed in various kinds of processing, and the results of processing or the like.

3. Functional Configuration

As shown in FIG. 8, the main program 710 executed as main processing (FIG. 10) is stored in the storage unit 700. The main program 710 includes, as sub-routines, an activity analysis program 722 for analyzing an action at the action analysis unit 115, and a reliability determination program 724 for determining the reliability of the measured pulse rate 750.

Also, the measured pulse rate 750, the estimated pulse rate 755, the SN value 770, and the action-specific pulse estimation table 780 are stored as data in the storage unit 700.

4. Flow of Processing

FIG. 10 is a flowchart showing the flow of the main processing (biological information processing method) executed in the pulsimeter 1 by the processing unit 100 reading out the main program 710 stored in the storage unit 700.

First, the processing unit 100 carries out initial setting (Step S1800). Specifically, various calculation parameters used in the main processing, and the values of the measured pulse rate 750, the estimated pulse rate 755 and the SN value 770 or the like stored in the storage unit 700 are initialized.

Next, the processing unit 100 acquires the result of detection by the pulse rate sensor 10 and the body motion sensor 20 (Step S1802).

Then, the processing unit 100 performs action analysis processing on the subject according to the action analysis program 722 stored in the storage unit 700 (Step S1830).

FIG. 11 is a flowchart showing the flow of the action analysis processing (activity information derivation process).

First, the action analysis unit 115 analyzes a body motion signal to extract acceleration data indicating the acceleration of a body motion and determines whether the acceleration data has periodicity or not (Step S1832).

In this Embodiment 2 , in the determination of periodicity of the acceleration data, fast Fourier transform (FFT) processing is performed on the acceleration data over a predetermined period of time and a frequency component is extracted from the result of the FFT processing on the acceleration data. Here, if a frequency component that is valid as a body motion signal is extracted from the frequency components, the periodicity of the acceleration is exhibited and therefore it is determined that the acceleration data has periodicity. Meanwhile, if a frequency component that is valid as a body motion signal is not extracted, it is determined that the acceleration data has no periodicity.

If it is determined that the acceleration data has periodicity as a result of the determination (Yes in Step S1834), the action analysis unit 115 acquires the magnitude of the moving rate (Step S1836). In this Embodiment 2, the period of the acceleration is calculated on the basis of the frequency of the frequency component that is valid as a body motion signal, and the moving rate is calculated as the number of steps per minute (average pace) based on the number of vibrations corresponding to the period.

Next, the action analysis unit 115 compares the calculated moving rate with a predetermined reference value (Step S1838), and if the calculated moving rate is greater than the predetermined reference value (Yes), the action analysis unit 115 determines that the action of the subject is "running" (Step S1840) and ends the action analysis processing.

Meanwhile, if the calculated moving rate is not greater than the predetermined reference value (No in Step S1838), the action analysis unit 115 determines that the action of the subject is "walking" (Step S1842) and ends the action analysis processing.

Also, if it is determined that the acceleration data has no periodicity (No in Step S1834) as a result of the determination in Step S1832 on whether the acceleration data has periodicity or not, the action analysis unit 115 performs power spectrum analysis on the acceleration data (Step S1844) .

In this Embodiment 2, the power held by each frequency component acquired by performing FFT processing on the acceleration data is calculated, thus generating a power spectrum. The power spectrum expresses signal intensity (spectrum value) of each frequency band with respect to the sum total of the energies (powers) held by the acceleration data.

Next, the action analysis unit 115 determines whether the number of occurrences of a frequency component with a predetermined intensity, that is, falling within a predetermined proportion to the maximum value of power, in the generated power spectrum, is greater than a reference number or not (Step S1846). If the number of occurrences is equal to or below the reference number (No in Step S1846), the action analysis unit 115 determines that the action of the subject is "calisthenics" (Step S1848) and ends the action analysis processing.

Meanwhile, if the number of occurrences is greater than the reference number (Yes in Step S1846), the action analysis unit 115 determines that the action of the subject is "other" (Step S1850) and ends the action analysis processing.

In this Embodiment 2, "walking", "running" and "calisthenics" are identif ied as action patterns, and the other actions are classified as "other". However, this classification is not limiting. For example, it is possible to consider an example in which "cycling", which is a non-periodical exercise that causes a rise in pulse rate, is further identified on the basis of the amount of noise components in the power spectrum.

Back to FIG. 10, the processing unit 100 acquires the result of the action analysis processing, and if the action of the subject is determined as "running" or "walking" (Yes in Step S1804), the processing unit 100 calculates the moving rate from the output signal of the body motion sensor 20 (Step S1810) <body motion information detection process>.

Then, the processing unit 100 estimates the estimated pulse rate 755 from the moving rate, referring to the action-specific pulse estimation table 780 corresponding to one of "walking" and "running" shown in FIG. 9 (Step S1812), and proceeds to Step S1816.

Meanwhile, if the action of the subject is determined as neither "running" nor "walking" (No in Step S1804) but as "calisthenics" (Yes in Step S1806), the processing unit 100 estimates estimated pulse rate 755 on the basis of the intensity of acceleration, referring to the action-specific pulse estimation table 780 corresponding to "calisthenics" shown in FIG. 9 (Step S1808), and proceeds to Step S1816.

Also, if the action of the subject is determined as "other" (No in Step S1806), the value displayed as the pulse rate of the subject in the previous measurement processing (previous value) is used as the estimated pulse rate 755 (Step S1814), and the processing goes to Step S1816. Although not illustrated, it is possible to consider an example in which an action-specific pulse estimation table 780 corresponding to "other" is prepared so that the estimated pulse rate 755 corresponding to "other" is estimated.

In Step S1816, the processing unit 100 starts pulse rate measurement processing of measuring the pulse rate of the subject, using the result of the detection of the pulse wave signal by the pulse rate sensor 10 and the result of the detection by the body motion sensor 2 0, and calculates the pulse rate from the result of the measurement <biological information detection process>, and stores the calculated pulse rate in the storage unit 700 as the measured pulse rate 750.

Next, the processing unit 100 performs reliability determination processing on the measured pulse rate 750 (Step S1860).

FIG. 12 is a flowchart showing the flow of the reliability determination processing (reliability determination process).

First, the reliability determination unit 160 determines whether the measured pulse rate 750 is within a predetermined range or not (Step S1862). The predetermined range is a preset range indicating an expected range of pulse rates. Here, if it is determined that the measured pulse rate 750 is within the predetermined range (Yes in Step S1862), the reliability determination unit 160 acquires the SN value 770 of the pulse wave signal (Step S1864).

The reliability determination unit 160 determines whether the SN value 770 satisfies a predetermined criterion or not (Step S1866). If the SN value 770 is determined as satisfying the predetermined criterion (Yes in Step S1866), the reliability determination unit 160 determines that the reliability of the measured pulse rate 750 is "high" (Step S1868) and ends the reliability determination processing.

Meanwhile, if it is determined in Step S1862 that the measured pulse rate 750 is not within the predetermined rage (No), or if it is determined in Step S1866 that the SN value 770 does not satisfy the predetermined criterion (No), the reliability determination unit 160 determines that the reliability of the measured pulse rate 750 is "low" (Step S1870) and ends the reliability determination processing.

Back to FIG. 10, the processing unit 100 determines whether the reliability determination unit 160 evaluates the reliability of the measured pulse rate 750 as "high" or not (Step S1818) <decision process>.

Here, if the reliability determination unit 160 evaluates the reliability of the measured pulse rate 750 as "high" (Yes in Step S1818), the processing unit 100 acquires the measured pulse rate 750 from the storage unit 700 as the pulse rate of the subject and displays the measured pulse rate 750 on the display unit 300 (Step S1820) <output process>, and proceeds to Step S1824.

Meanwhile, if the reliability of the measured pulse rate 750 is evaluated as "low" (No in Step S1818), the processing unit 100 acquires the estimated pulse rate 755 from the storage unit 700 as the pulse rate of the subject, then displays the estimated pulse rate 755 on the display unit 300 (Step S1822), and proceeds to Step S1824.

In Step S1824, the processing unit 100 holds the pulse rate displayed on the display unit 300, as a previous value. Next, the processing unit 100 determines whether to end the main processing or not (Step S1826). If the main processing is not to end (No in Step S1826), the processing unit 100 returns to Step S1802.

Meanwhile, if the main processing is to end (Yes in Step S1826), the processing unit 100 ends the main processing.

The above-described Embodiment 2 has the following effects.

(1) The action-specific pulse estimation table 780 used to estimate pulse rate is prepared for each action, and the action analysis unit 115 analyzes the action of the subject and decides the action-specific pulse estimation table 780 to be applied. Therefore, since the estimated pulse rate 755 is estimated with reference to the action-specific pulse estimation table 780 corresponding to the action of the subject, the estimation accuracy of the estimated pulse rate 755 can be improved.

(2) The reliability determination unit 160 determines the reliability of the measured pulse rate 750 calculated on the basis of the pulse wave signal detected by the pulse wave sensor 10. If the reliability is determined as high, the measured pulse rate 750 is displayed as the pulse rate of the subject, whereas if the reliability is determined as low, the estimated pulse rate 755 is displayed as the pulse rate of the subject. Therefore, since the estimated pulse rate 755 is displayed even if the measured pulse rate 750 is not correctly measured for some reason, the convenience and reliability of the pulsimeter 1 can be improved.

(Embodiment 3)

Next, Embodiment 3 of the invention will be described with reference to FIGS. 13 to 16.

(Principles)

A pulsimeter 1 to which the biological information processing device of the invention is applied calculates the pulse rate of the subject, using a signal capturing change in the amount of bloodstream detected by a pulse wave sensor 10 (hereinafter referred to as a bloodstream amount signal). The change in the amount of bloodstream is influenced by the pulsation due to pulses, and the body motion of the subject such as the moving rate or swings of the arms. Therefore, the bloodstream amount signal is a signal made up of a pulsation component signal and a body motion component signal of the subject superimposed on each other. The pulsimeter 1 eliminates the body motion component signal from the bloodstream amount signal and thus extracts the pulsation component signal, using a body motion signal outputted from a body motion sensor 20.

Specifically, the pulsimeter 1 includes a digital filter such as an FIR (finite impulse response) filter, for example, as an adaptive filter, and executes processing of eliminating the body motion noise component from the bloodstream amount signal as digital signal processing, using the adaptive filter. Moreover, the pulsimeter 1 performs frequency resolution processing on the extracted pulsation component signal and thus extracts a signal intensity value (spectrum value) of each frequency band. As the frequency resolution processing, for example, FFT (fast Fourier transform) can be employed. The pulsimeter 1 specifies a frequency spectrum equivalent to the pulse wave of the subject, from the extracted signal intensity value, and finds the pulse rate on the basis of the frequency (or period) thereof. The pulsimeter 1 calculates the pulse rate at a predetermined time interval (for example, at an interval of 1 to 5 seconds). In the description below, the pulse rate calculated as described above is called a "measured pulse rate".

The pulsimeter 1 displays the result of measuring the measured pulse rate on a display panel 4 as the pulse rate and thus notifies the subject. By the way, there are cases where the pulsimeter 1 may calculate a measured pulse rate that deviates from the actual pulse rate of the subject. This is because the blood circulation on the surface of a human body can get worse depending on the state at the time of measurement, such as in measurement in an environment with low ambient temperature or measurement when the subject suddenly begins exercising. In such cases, since the bloodstream amount signal weakens, the amount of information of the bloodstream amount signal decreases and the calculation accuracy of the adaptive filter processing and the frequency resolution processing falls, thus affecting the accuracy of the measured pulse rate. If the measured pulse rate that is calculated deviates from the actual pulse rate of the subject, the resulting measured pulse rate is an abnormal value and it is not appropriate to notify the subject of the value. Therefore, if the measured pulse rate is not appropriate as a result of determining whether the measured pulse rate is appropriate or not, the pulsimeter 1 needs to notify the subject of an appropriate pulse rate.

(Pulse Rate Calculation Processing When State of Exercise Changes)

The processing of calculating the measured pulse rate when the state of exercise of the subject changes will be described, using FIG. 13.

FIG. 13 is a graph showing pulse rates when the state of exercise changes under limiting conditions. The limiting conditions are obtained by analyzing the phenomenon of the measured pulse rate differing from the actual pulse rate of the subject, and then narrowing down and deriving states, actions, movement and the like of the subject that are necessary for the phenomenon to occur. For example, conditions include the degree of detectability of the amount of bloodstream of the subject, the pulse rate before the start of exercise, the pulse rate immediately after the start of exercise, the type of exercise, and the like. With these limiting conditions, if a specific relation between the actual pulse rate of the subject and the period of the movement of the subject is generated, the phenomenon is likely to occur. The graph of FIG. 13 is an example of this, showing data obtained by applying the limiting conditions to an actual subject and reproducing the phenomenon. The above limiting conditions are analyzed and derived on the basis of experiment data on a plurality of subjects accumulated in advance.

The horizontal axis of the graph in FIG. 13 represents the time elapsed (minute), and the vertical axis represents the pulse rate (bpm (beat per minute)) or the value of the moving rate (steps/minute). The subject begins exercising (running) at the timing of 0 minutes and continues running for the time elapsed of 2 minutes. During this time, the pulsimeter 1 measures and calculates the actual pulse rate, the measured pulse rate, an estimated pulse rate (described later) and the moving rate of the subject at every predetermined time interval, and plots these as an actual pulse rate L1 (solid line), a measured pulse rate L2 (chain dotted line), an estimated pulse rate L3 (dashed line), and a moving rate L4 (chain double-dotted line). The measured pulse rate L2 shows values close to the actual pulse rate L1 during the period of 0 to 0.2 minutes. The part where the chain dotted line is interrupted during the period from 0.2 minutes to 0.4 minutes indicates that the pulse rate cannot be calculated because of the influence of noise or the like.

It can be understood that the subject runs at a stable pace with the value of the moving rate within the range of 180 to 200 (steps/minute) during the period of 0 to 2 minutes, as shown by the moving rate L4. The actual pulse rate L1 is about 80 bpm at the start of exercise and gently increases to about 165 bpm after the lapse of 2 minutes. The measured pulse rate L2 is about 80 bpm at the start of exercise, then increases in a curve similar to the actual pulse rate L1 up to 0.2 minutes, but then falls to about 90 bpm after 0.4 minutes, and has values within the range of about 90 to 100 bpm until 2 minutes. Since the measured pulse rate L2 has values that largely deviate from the actual pulse rate L1 during the period of 0.4 to 2 minutes, it is hard to say that appropriate pulse rates are calculated.

How the measured pulse rate L2 is calculated as inappropriate values will be explained. First, the body motion signal component is eliminated from the bloodstream amount signal detected by the pulse wave sensor 10. The body motion signal component represents the periodical movement indicated by the moving rate L4 and therefore a component of 180 to 200 (steps/minute) as a frequency component. The frequency component of 180 to 200 (steps/minute) and a frequency component of 90 to 100 (steps/minute) which appears due to the influence of the above frequency component and which is about half the above frequency component should be eliminated from the bloodstream amount signal, and the pulsation component signal should be left as a principal component. However, since the pulsation component signal changes with a tendency to increase between about 80 and 160 bpm, the period is not stable.

Moreover, because of the worsening of the blood circulation on the body surface due to the sudden start of exercise by the subject, the pulsation component signal does not emerge as a spectrum value that is intense enough to be discriminated as a principal component. Also, in such a circumstance, signal components that cannot be eliminated remain near the frequency component of 90 to 100 (steps/minute), which is about half the body motion signal component, and emerge as a relatively intense spectrum value. Since the measured pulse rate L2 at the start of exercise increases from about 80 bpm, the frequency component of 90 to 100 (steps/minute) is grasped as a continuation of the pulsation signal component and therefore calculated as the measured pulse rate. In this way, the measured pulse rate L2 is calculated as 90 to 100 bpm during the period of 0.4 to 2.0 minutes. Also, if there is only a small difference in the intensity of the spectrum value between the half component of the body motion signal component and the changing pulsation signal component, it may result in the inability to specify the pulse rate as indicated by the measured pulse rate L2 during the period of 0.2 to 0.4 minutes.

In this way, there are cases where the inappropriate measured pulse rate L2 may be calculated. Therefore, it is necessary to determine whether the measured pulse rate is appropriate or inappropriate.

Next, the determination on whether the measured pulse rate is appropriate or not will be explained.

Whether the measured pulse rate is appropriate or not is determined by a first determination unit and a second determination unit. The first determination unit determines that the measured pulse rate is likely to be inappropriate, if the difference between the measured pulse rate and a pulse rate estimated on the basis of the body motion information of the subject departs from a predetermined allowable range. The second determination unit determines that the measured pulse rate is likely to be inappropriate if the value of the ratio of the signal component of the measured pulse rate to the other frequency components is below a predetermined threshold, in the frequency resolution processing. The pulse rate estimated on the basis of the body motion information in the first determination unit is called an "estimated pulse rate", and the predetermined allowable range is called a "window". Also, the value of the ratio of the signal component (signal) determined as the measured pulse rate to the other frequency components (noise) in the second determination unit is called an "SN ratio (signal to noise ratio)".

(Calculation of Estimated Pulse Rate)

The estimated pulse rate is a pulse rate calculated using the body motion information of the subject and is found from the correlation between physical exercise intensity found from a physical action such as exercise and the pulse rate appearing in the subject. A preferred example of the scale of the physical exercise intensity is the moving rate (steps/minute) of the subject. That moving rate is calculated from the acceleration detected by the body motion sensor 20 as described above and is calculated every unit time (for example, at an interval of 1 to 5 seconds). The method for calculating the estimated pulse rate from the moving rate is disclosed, for example, in JP-A-2012-232010. At the time of exercise, the actual pulse rate increases relatively quickly from the pulse rate before the start of exercise, and changes to converge to a predetermined pulse rate (target pulse rate) when the moving rate of the subject is maintained. The target pulse rate is calculated from the value of the moving rate, using an approximation formula.

At the start of exercise of the subject, the calculation of an estimated pulse rate according to an exercise start model is applied. The pulse rate is formulated in such a way that as the time elapsed after the start of exercise becomes longer, the pulse rate increases in the way of logarithmic function and gradually approaches the target pulse rate. That is, the estimated pulse rate at the start of exercise changes from the pulse rate before the start of exercise to the target pulse rate and is thus found as the pulse rate corresponding to the time elapsed.

At the end of exercise, the calculation of an estimated pulse rate according to an exercise end model is applied. The estimated pulse rate can be defined and calculated by a calculation formula in which the estimated pulse rate decreases sigmoidally from the target pulse rate to the pulse rate after the end of exercise. That is, the estimated pulse rate at the end of exercise changes from the target pulse rate to the pulse rate after the end of exercise and is thus found as the pulse rate corresponding to the time elapsed.

When the subject is neither at the start of exercise nor at the end of exercise, the subject is at rest or during exercise except the start of exercise and the end of exercise. In such a case, the target pulse rate found from the moving rate is the estimated pulse rate.

While the target pulse rate is described as being calculated from the value of the moving rate by an approximation formula, the target pulse rate may be derived from a correlation table between moving rate and target pulse rate, prepared for each subject. More specifically, the correlation table between moving rate and target pulse rate is prepared, taking information such as the athletic ability of the subject and the state during exercise into account. Moreover the correlation table may be updated according to need, on the basis of the latest information of the exercise and pulse rate of the subject that are measured. By thus using the target pulse rate taking the athletic ability of the subject and the state during exercise into account, it is possible to calculate an estimated pulse rate with high accuracy to the actual pulse rate of the subject.

Also, the target pulse rate may be derived from a correlation table between moving rate and target pulse rate, prepared by analyzing action patterns. More specifically, a correlation table between moving rate and target pulse rate prepared, taking exercise intensity varying from one action pattern to another (walking, running and the like) into account, is used. The correlation table is statistically analyzed and prepared, using experiment data that are accumulated in advance. The body motion information acquired from the body motion sensor 20 is analyzed to specify the action pattern of the subject. Using the correlation table corresponding to the action pattern of the subject, the target pulse rate is acquired from the moving rate. By thus specifying the action pattern and acquiring the target pulse rate, it is possible to calculate an estimated pulse rate with high accuracy based on the exercise intensity corresponding to the action pattern.

(Window Processing)

Next, the window used as the measure for determination in the first determination unit will be explained.

The window is a range where the actual pulse rate of the subject is likely to exist. Since the estimated pulse rate is a pulse rate estimated from the body motion information, it is difficult to make the estimated pulse rate coincide accurately with the actual pulse rate of the subject. Thus, a window width where the actual pulse rate of the subject is likely to exist is provided in the high-low direction of the estimated pulse rate. Whether the calculated estimated pulse rate is within the window width or not is determined, and whether the measured pulse rate is appropriate or inappropriate is determined. As the window width, a predetermined range from the estimated pulse rate may be provided. For example, the window width may be fixed to ±10 beats or the like. Also, as another example, a configuration in which the value of the window width is stored for each moving rate, in the correlation table between moving rate and target pulse rate.

(Application of Window Processing)

FIG. 14 is a graph showing the application of the window processing, extracting and explaining a part of the graph shown in FIG. 13. As in FIG. 13, the actual pulse rate L1 (solid line), the measured pulse rate L2 (chain dotted line) and the estimated pulse rate L3 (dashed line) are plotted. Also, the measured pulse rate and the estimated pulse rate that are calculated are plotted at each timing of calculation, with black dots indicating the measured pulse rate and black squares indicating the estimated pulse rate. The arrows extending up and down from the estimated pulse rate indicate the window width. Here, the window width is fixed at ±10 beats.

The measured pulse rate L2 is calculated to be 80 to 100 bpm during the period of 0.4 to 2.0 minutes, whereas the estimated pulse rate L3 gradually approaches the vicinity of the pulse rate of 170 beats with the lapse of time, drawing a mountain-shaped curve. The estimated pulse rate L3 has a curved shape with substantially the same tendency as the actual pulse rate L1 during the time elapsed of 0 to 2.0 minutes and shows numerical values closer to the actual pulse rate L1 than the measured pulse rate L2.

The window width of the estimated pulse rate L3 does not include the measured pulse rate L2 during the period of 0.4 to 2.0 minutes. Therefore, it is determined that the measured pulse rate L2 is likely to be an inappropriate pulse rate.

In this way, whether the measured pulse rate that is calculated is appropriate or not can be determined, using the window based on the estimated pulse rate estimated from the body motion information.

(Calculation of S/N Ratio)

Next, the SN ratio used as the measure for determination by the second determination unit will be explained.

The SN ratio is the value of the ratio of the signal component determined as the measured pulse rate (signal) to the other frequency components (noise) and is a scale for indicating the reliability of the measured pulse rate that is calculated. As the value of the SN ratio increases, the reliability of the measured pulse rate that is calculated rises. The calculation of the SN ratio is carried out as follows, for example.

Digital signal processing is performed on a bloodstream amount signal that is detected and frequency resolution processing is performed on a pulsation component signal thus extracted. Then, a baseline with a maximum spectrum value is selected and the baseline is defined as the baseline of a measure pulse rate component. Also, of the baselines excluding baselines included in a predetermined range near the maximum baseline, for example, a baseline with the second largest spectrum value is selected as a noise baseline.

Then, using the spectrum value Ps of the baseline of the measured pulse rate component and the spectrum value Pn of the noise baseline, Ps is divided by Pn to calculate the SN ratio.

In a circumstance where baselines with large spectrum values exit alongside each other, making it difficult to discriminate the signal component from the noise component, the reliability of the measured pulse rate calculated on the basis of the result of frequency resolution processing falls. In such a circumstance, the SN ratio tends to be low. Thus, a threshold Pr for the SN ratio is defined, and if the SN ratio is above the threshold Pr, the reliability of the measured pulse rate that is calculated is determined as high. The threshold Pr is calculated on the basis of the result of statistical analysis from the value of the SN ratio in the case where the measured pulse rate that is calculated results turns out to be close to the actual pulse rate from the experiment data that is accumulated.

Also, by varying the threshold Pr within a predetermined range, it is possible to tighten or loosen the reliability determination on the measured pulse rate. If the reliability determination is to be tightened, the threshold Pr is set to be greater than in the case of loosening. The threshold Pr is equivalent to the predetermined reference value.

(Functional Configuration)

FIG. 15 is a block diagram showing an example of the functional configuration of the pulsimeter. The pulsimeter 1 includes the pulse wave sensor 10, the body motion sensor 20, a pulse wave signal amplifying circuit unit 30, a pulse waveform shaping circuit unit 40, a body motion signal amplifying circuit unit 50, a body motion waveform shaping circuit unit 60, an A/D (analog/digital) conversion unit 70, a processing unit 100, an operation unit 200, a display unit 300, a notification unit 400, a communication unit 500, a clock unit 600, and a storage unit 700.

The pulse wave sensor 10 is a sensor which measures pulse waves of the subject with the pulsimeter 1 installed thereon, and is configured with a photoelectric pulse wave sensor, for example. The pulse wave sensor 10 detects a change in volume caused by an influx of the bloodstream into body tissues, as a pulse wave signal, and outputs the pulse wave signal to the pulse wave signal amplifying circuit unit 30.

The pulse wave signal amplifying circuit unit 30 is an amplifying circuit which amplifies the pulse wave signal inputted from the pulse wave sensor 10, with a predetermined gain. The pulse wave signal amplifying circuit unit 30 outputs the amplified pulse wave signal to the pulse waveform shaping circuit unit 40 and the A/D conversion unit 70.

The pulse waveform shaping circuit unit 40 is a circuit unit which shapes the pulse wave signal amplified by the pulse wave signal amplifying circuit unit 30 and is configured with a circuit for eliminating high-frequency noise components, a clipping circuit, and the like. The processing unit 100 determines whether pulse waves are detected or not, on the basis of the pulse waveform shaped by the pulse waveform shaping circuit unit 40.

The body motion sensor 20 is a sensor for capturing motions of the subject with the pulsimeter 1 installed thereon, and is configured at least with an acceleration sensor.

The body motion signal amplifying circuit unit 50 is an amplifying circuit which amplifies the body motion signal inputted from the body motion sensor 20, with a predetermined gain. The body motion signal amplifying circuit unit 50 determines whether a body motion is detected or not, on the basis of the body motion waveform of the amplified body motion signal shaped by the body motion waveform shaping circuit unit 60.

The body motion waveform shaping circuit unit 60 is a circuit unit which shapes the body motion signal amplified by the body motion signal amplifying circuit unit 50 and is configured with a circuit for eliminating high-frequency noise components, a circuit for discriminating a gravitational acceleration component from the other components, a clipping circuit, and the like. The processing unit 100 determines whether a body motion is detected or not, on the basis of the body motion waveform shaped by the body motion waveform shaping circuit unit 60.

The A/D conversion unit 70 samples and digitizes the pulse wave signal in an analog format amplified by the pulse wave signal amplifying circuit unit 30 and the body motion signal in an analog format amplified by the body motion signal amplifying circuit unit 50, each at a predetermined sampling interval, and thus converts these signals into digital signals. Then, the A/D conversion unit 70 outputs the converted digital signals to the processing unit 100.

The processing unit 100 is a control device and computing device which centrally control each part of the pulsimeter 1 according to various programs such as a control program stored in the storage unit 700, and has a processor such as a CPU (central processing unit) or DSP (digital signal processor). The processing unit 100 performs pulse rate calculation processing according to a pulse rate calculation program 732 stored in the storage unit 700, and performs control to measure or estimate the pulse rate of the subject with the pulsimeter 1 installed thereon, decide a pulse rate that is suitable as the pulse rate of the subject, and cause the display unit 300 to display the pulse rate.

The processing unit 100 has a pulse rate measurement unit (measured pulse rate calculation unit) 110, a moving rate calculation unit 120, an estimated pulse rate calculation unit 132, a window setting unit 134, a window processing determination unit 136, an SN ratio calculation unit 142, an SN determination unit 144, a pulse rate decision unit 146, and a display control unit 148, as functional units. However, these functional units are simply an example and not all the functional units need to be essential components.

The measured pulse rate calculation unit 110 calculates the measured pulse rate from the bloodstream amount signal measured by the pulse wave sensor 10. More specifically, the measured pulse rate calculation unit 110 eliminates the body motion noise component from the bloodstream amount signal inputted via the A/D conversion unit 70 and thus extracts the pulsation component signal. The measured pulse rate calculation unit 110 performs frequency resolution processing on the pulsation component signal, analyzes the signal intensity value of each frequency, and specifies a frequency spectrum corresponding to pulse waves. The measured pulse rate calculation unit 110 calculates the pulse rate (measured pulse rate) from the frequency of the frequency spectrum of the pulse waves. The measured pulse rate calculation unit 110 is equivalent to the detection unit which detects a measured pulse rate on the basis of the biological information of the subject.

The moving rate calculation unit 120 calculates the moving rate as the body motion information, from the body motion signal measured by the body motion sensor 20. More specifically, the frequency spectrum of a periodical body motion signal is extracted from the body motion signal (body motion data) inputted from the A/D conversion unit 70, and moving rate is calculated from the frequency of the frequency spectrum.

The estimated pulse rate calculation unit 132 has a gradual convergence estimation unit (not shown) which finds a target pulse rate using the moving rate and finds the estimated pulse rate by causing the pulse rate to gradually approach the target pulse rate with the lapse of time from the pulse rate measured before a change in the state of exercise by the measured pulse rate calculation unit 110. The target pulse rate is found from a subject target pulse rate table 748 or action-specific target pulse rate table 752 taking correspondence between moving rate and target pulse rate.

The window setting unit 134 sets a window width 742 for the window processing determination unit 136 to determine whether a measured pulse rate 734 is appropriate or not, using an estimated pulse rate 755. The window width 742 is a preset fixed value. However, the window width 742 may be set for each target pulse rate or may be derived by a computing formula or the like.

The window processing determination unit 136 determines whether the measured pulse rate 734 calculated by the measured pulse rate calculation unit 110 is appropriate or not, using the window set by the window setting unit 134. The window processing determination unit 136 is equivalent to the first determination unit.

The SN threshold setting unit 138 sets the value of an SN threshold 746 as a predetermined reference value, on the basis of the result in the window processing determination unit 136 on whether the measured pulse rate 734 that is calculated is appropriate or not.

As the value of the SN threshold 746 in the case where the measured pulse rate 734 is determined as appropriate, a smaller value than the value of the SN threshold 746 in the case where the measured pulse rate 734 is determined as inappropriate is set. The SN threshold setting unit 138 is equivalent to the reference value calculation unit.

The SN ratio calculation unit 142 calculates an SN ratio 744 by analyzing the frequency characteristic including the frequency spectrum generated by the frequency resolution processing on the pulsation component signal in the measured pulse rate calculation unit 110. The SN ratio 744 is equivalent to the reliability degree information of the measured pulse rate. The SN ratio calculation unit 142 is equivalent to the reliability degree information calculation unit.

The SN determination unit 144 compares the SN ratio 744 calculated by the SN ratio calculation unit 142 on the basis of the SN threshold 746. If the value of the SN ratio 744 is above the SN threshold 746, the measured pulse rate 734 that is calculated is determined as having high reliability. If the SN ratio 744 is equal to or below the SN threshold 746, the measured pulse rate 734 that is calculated is determined as having low reliability. The SN determination unit 144 is equivalent to the second determination unit.

The pulse rate decision unit 146 decides which of the measured pulse rate 734 and the estimated pulse rate 755 that are calculated should be reported to the subject, on the basis of the results of the determination by the window processing determination unit 136 and the SN determination unit 144. The pulse rate decision unit 146 stores the pulse rate that is decided, in the storage unit 700 as a decided pulse rate 738. The pulse rate decision unit 146 is equivalent to the decision unit.

The display control unit 148 performs display control on the display unit 300 to display the pulse rate on the basis of the result of the decision by the pulse rate decision unit 146.

The operation unit 200 is an input device configured with button switches or the like and outputs the signal of a button that is pressed, to the processing unit 100. With operations on this operation unit 200, various instructions such as a pulse measurement instruction are inputted. The operation unit 200 is equivalent to the operation buttons 5 in FIG. 1. The configuration of the operation unit 200 is not limited to this and may be any configuration that enables a plurality of operation inputs. The display panel 4 may have a touch panel function.

The display unit 300 is a display device which is configured with an LCD (liquid crystal display) or the like and which performs various displays based on display signals inputted from the processing unit 100. Various kinds of information about a living body (pulse rate, exercise intensity, calories burned and the like) are displayed on the display unit 300. The display unit 300 is equivalent to the display panel 4 in FIG. 1.

The notification unit 400 is a notification device which is configured with a speaker, a piezoelectric oscillator and the like and which issues various notifications based on notification signals inputted from the processing unit 100. For example, the notification unit 400 outputs an alarm sound from the speaker or causes the piezoelectric oscillator to oscillate, thus issuing various notifications to the subject.

The communication unit 500 is a communication device for transmitting and receiving information utilized inside the device, to and from an external information processing device such as a personal computer (PC), under the control of the processing unit 100.

As the communication method of this communication unit 500, various methods can be applied, such as a method of wired connection via a cable conforming to a predetermined communication standard, a method of connection via an intermediate device which also serves as a charger, called a cradle, and a method of wireless connection using near field radio communication.

The clock unit 600 is configured with a crystal oscillating device made up of a crystal oscillator and an oscillation circuit, and has the clock function of the pulsimeter 1, the stopwatch function, and the time measurement function such as generating a sampling time for detection of biological information and body motion information. The time measured by the clock unit 600 is constantly outputted to the processing unit 100.

The storage unit 700 is configured by a storage device such as a ROM (read only memory), flash ROM, or RAM (random access memory), and stores the control program of the pulsimeter 1 and various programs and data or the like for realizing various functions such as the pulse rate measurement/estimation function, the exercise intensity measurement function, and the calorie measurement function. Also, the storage unit 700 has a work area for temporarily storing data currently being processed in various kinds of processing, and the results of processing or the like.

In the storage unit 700, the pulse rate calculation program 732 executed by the processing unit 100 as the pulse rate calculation processing is stored as a program. Also, in the storage unit 700, the measured pulse rate 734, the estimated pulse rate 755, the decided pulse rate 738, the window width 742, the SN ratio 744 and the SN threshold 746 are stored as data. Moreover, in the storage unit 700, the subject target pulse rate table 748 and the action-specific target pulse rate table 752 are housed (stored) as data tables.

(Flow of Processing)

FIG. 16 is a flowchart showing the flow of the pulse rate decision processing. Hereinafter, the flow is explained mainly with reference to FIG. 16 and also to FIG. 15 according to need. The following flow is equivalent to the biological information measurement method and is executed by the processing unit 100 controlling the respective parts including the storage unit 700 on the basis of the pulse rate calculation program 732 stored in the storage unit 700. Also, by the execution of the pulse rate calculation program 732, the functions of the respective functional units including the measured pulse rate calculation unit 110 included in the processing unit 100 are realized.

In Step S2010, preparations for the calculation of the measured pulse rate 734 and the calculation of the moving rate 736 are carried out. More specifically, first, a timer is set using the real-time clock of the clock unit 600. The timer sets at least the sampling period of the pulse wave sensor 10 and the body motion sensor 20. Also, pulse wave data and body motion data detected using the sampling period are accumulated and a predetermined period of time for calculating the measured pulse rate 734, the moving rate 736 and the estimated pulse rate 755 is set. As for the sampling period, for example, sampling is performed 16 to 64 times per second and detection is performed at an interval of about 15.6 to 62.5 msec. The predetermined period of time is set, for example, to approximately 1 to 5 seconds. This is the processing repeated every predetermined period of time in question, in Steps S2020 to S2130 below. That is, after measurement is started by the subject, while pulse waves and body motions are detected by the pulse wave sensor 10 and the body motion sensor 20, the pulse rate is decided and reported to the subject at the timing of once every 1 to 5 seconds.

In Step S2020, the measured pulse rate 734 is calculated. More specifically, elimination of the body motion noise component and frequency resolution processing are carried out on the bloodstream amount signal corresponding to the predetermined period of time (t seconds) measured by the pulse wave sensor 10. The frequency spectrum corresponding to pulse waves is specified and the measured pulse rate 734 is calculated. The result of this is stored in the storage unit 700 as the measured pulse rate 734. Steps 2020 is equivalent to the detection process.

In Step S2030, the moving rate 736 is calculated. More specifically, the frequency spectrum of a periodical body motion signal is extracted from the body motion signal corresponding to the t seconds measured by the body motion sensor 20, and the moving rate 736 is calculated. The result of this is stored in the storage unit 700 as the moving rate 736. Step S2030, and Step S2040 described below, are equivalent to the estimation process.

In Step S2040, the estimated pulse rate 755 is calculated. More specifically, a target pulse rate is found from the subject target pulse rate table 748 or the action-specific target pulse rate table 752, using the moving rate calculated in Step S2030. Also, the time of occurrence of the moving rate is acquired from the stored moving rate 736 and the pulse rate decided immediately before that time is acquired from the decided pulse rate 738. On the basis of the pulse rate immediately before the time of occurrence of the moving rate, the time elapsed from the time of occurrence of the moving rate, and the target pulse rate, the estimated pulse rate 755 is found by the gradual convergence estimation unit (not shown).

In Step S2050, the window width 742 based on the estimated pulse rate 755 is calculated. More specifically, a predetermined range of the window width 742 is set to ±10 beats on the basis of the estimated pulse rate 755 calculated in Step S2040, as a reference. Step S2050 is included in the first determination process.

In Step S2060, whether the measured pulse rate 734 is within the range of the window width 742 or not is compared. If the measured pulse rate 734 is (the estimated pulse rate - 10) or above and (the estimated pulse rate + 10) or below (Yes), the processing goes to Step S2070. If the measured pulse rate 734 is below (the estimated pulse rate - 10) or above (the estimated pulse rate + 10) (No), the processing goes to Step S2080. Step S2060 is equivalent to the first determination process.

In Step S2070, the SN threshold 746 for loosening the SN determination criterion is calculated. More specifically, if the SN ratio 744 is above the SN threshold Pr, the reliability of the measured pulse rate 734 that is calculated is likely to be high, as described above. Here, if the SN threshold 746 is set to a smaller value than Pr, the SN determination criterion is loosened. Specifically, for example, the SN threshold 746 is set to a value 0.8 times Pr.

In Step S2080, the SN threshold 746 for tightening the SN determination criterion is calculated. More specifically, if the SN threshold 746 is set to a larger value than Pr, the SN determination criterion is tightened. Specifically, for example, the SN threshold 746 is set to a value 1.2 times Pr.

In Step S2090, the SN ratio 744 of the measured pulse rate 734 is calculated. More specifically, using the spectrum value Ps of the baseline of the measured pulse rate component and the spectrum value Pn of the noise baseline, Ps is divided by Pn. Step S2090 is equivalent to the calculation process.

In Step S2100, whether the SN ratio 744 of the measured pulse rate 734 is greater than the SN threshold 746 or not is compared. More specifically, the SN ratio 744 calculated in Step S2090 is compared with the SN threshold 746 calculated in Step S2070 and Step S2080. If the SN ratio 744 of the measured pulse rate 734 is greater (Yes), the reliability of the measured pulse rate 734 that is calculated is high and the processing goes to Step S2110. If the SN ratio 744 of the measured pulse rate 734 is equal or smaller (No), the reliability of the measured pulse rate 734 is low and the processing goes to Step S2120. Step S2100 is equivalent to the second determination process.

In Step S2110, the measured pulse rate 734 is decided as the pulse rate of the subject. The measured pulse rate 734 is determined as appropriate as the pulse rate of the subject and stored in the storage unit 700 as the decided pulse rate 738. Step S2110 and Step S2120 are equivalent to the decision process.

In Step S2120, the estimated pulse rate 755 is decided as the pulse rate of the subject. The measured pulse rate 734 is determined as inappropriate as the pulse rate of the subject, and the estimated pulse rate 755 is stored in the storage unit 700 as the decided pulse rate 738.

In Step S2130, whether to end the pulse measurement or not is determined. More specifically, the processing unit 100 ends the pulse rate calculation program 732 if the operation button 5 to the effect of ending the measurement is pressed by the subject (Yes) during the period of the above Steps S2020 to S2120. If the operation button 5 is not pressed (No), the processing unit 100 proceeds to Step S2020 and carries out the next pulse rate calculation.

(Advantageous Effects)

The window processing determination unit 136 determines the measured pulse rate 734 that is calculated, using the estimated pulse rate 755 based on the moving rate 736 by the moving rate calculation unit 120. The estimated pulse rate 755 is a pulse rate corresponding to the exercise load caused by change in the state of exercise and therefore follows the pulse rate of the subject that changes in a short period of time. By confirming whether or not the measured pulse rate 734 is within the range of the window width 742 indicating the state of deviation from the estimated pulse rate 755 with respect to whether the measured pulse rate 734 is appropriate or not, it is possible to determine whether the measured pulse rate 734 successfully follows the pulse rate that changes within a short period of time. That is, if the measured pulse rate 734 that is detected is included within a predetermined range including the estimated pulse rate 755, it means that the measured pulse rate 734 varies according to the body motion information and therefore can be determined as an appropriate pulse rate.

The SN determination unit 144 carries out determination on the basis of the SN ratio 744, which is the reliability degree information of the measured pulse rate 734. The SN ratio 744 is the reliability degree information that enables determination on whether the bloodstream amount signal acquired from the pulse wave sensor 10 includes little noise and intense pulse wave signals or not. As the SN ratio 744 is compared with the SN threshold 746 set by the SN threshold setting unit 138, it can be determined that the measured pulse rate 734 is appropriate if reliability is high.

The pulse rate decision unit 146 decides one of the measured pulse rate 734 and the estimated pulse rate 755 as an appropriate pulse rate, in consideration of both of the results of the determination by the window processing determination unit 136 and the SN determination unit 144.

In this way, the determination can be carried out using the information of both of the determination based on the body motion information of the subject (first determination unit) and the determination based on the biological information of the subject (second determination unit). The volume of information used for the determination is greater than in the determination using one of them, and even in a situation where the state of exercise of the subject changes, the pulse rate changing according to the body motion information can be decided with high accuracy and the subject can be notified of the correct pulse rate.

(Embodiment 4)

FIG. 17 is a flowchart showing the flow of pulse rate decision processing in Embodiment 4. In Embodiment 4, a part of the flowchart showing the flow of the pulse rate decision processing in Embodiment 3 (FIG. 16) is different. The flow below is equivalent to the biological information measurement method and is executed by the processing unit 100 controlling the respective parts including the storage unit 700 on the basis of the pulse rate calculation program 732 stored in the storage unit 700 shown in FIG. 15. Also, by the execution of the pulse rate calculation program 732, the functions of the respective functional units including the measured pulse rate calculation unit 110 included in the processing unit 100 are realized.

In Embodiment 3, Step S2100 as the second determination process is executed after Step S2060 as the first determination process is executed. However, this Embodiment 4 is different in that Step S2100 and Step 2060 are executed in this order. Also, Step S2070 and Step S2080 executed in Embodiment 3 are not carried out.

In Step S2100, whether the SN ratio 744 of the measured pulse rate is greater than the SN threshold 746 or not is compared. Pr is used as the SN threshold 746. If the SN ratio 744 of the measured pulse rate is greater (Yes), the reliability of the measured pulse rate that is calculated is high and the processing goes to Step S2060. If the SN ratio 744 of the measured pulse rate is equal or smaller (No), the reliability of the measured pulse rate is low and the processing goes to Step S2120.

In Step S2060, whether the measured pulse rate is within the range of the window width 742 or not is compared. If the measured pulse rate is (the estimated pulse rate - 10) or above and (the estimated pulse rate + 10) or below (Yes), the measured pulse rate that is calculated is determined as appropriate and the processing goes to Step S2110. If the measured pulse rate is below (the estimated pulse rate - 10) or above (the estimated pulse rate + 10) (No), the measured pulse rate is determined as inappropriate as the pulse rate of the subject and the processing goes to Step S2120. In Step S2120, the estimated pulse rate 755 is decided as the decided pulse rate 738.

As described above, the pulsimeter 1 according to this Embodiment 4 can achieve the following effects in addition to the effects in Embodiment 3.

Since Step S2100 is carried out before Step S2060, the processing of Step S2070 and Step S2080 can be omitted. Moreover, since Step S2100 and Step S2060 do not use variables depending on each other, the processing can be executed in parallel. Thus, as the time of the steps omitted in the pulsimeter 1 is reduced and the use of a multitasking structure enables parallel processing, the time taken for the pulse rate decision processing can be reduced. That is, while pulse rate is traditionally measured at an interval of 1 to 5 seconds, it is possible to measure and calculate pulse rate at a shorter interval.

Although the embodiments of the invention have been described with reference to the drawings, the specific configurations are not limited to the embodiments and include changes in setting or the like without departing from the scope of the invention.

For example, the configuration in which the pulse rate corresponding to the moving rate is estimated from the user-specific pulse estimation table 780 is not limiting. It is also possible to consider a configuration in which data of the moving rate and pulse rate of each subject are stored in the user-specific pulse estimation table 780, then the approximation formula of the following equation (1), which is a pulse estimation formula indicating the correlation between moving rate and pulse rate, is calculated from these data, and the pulse rate is estimated on the basis of the calculated approximation formula.

[Math. 1]

$$HR(p) = A*p^2+B*p+C \qquad \ldots (1)$$

Here, "p" is the moving rate and "HR(p)" is the pulse rate estimated from the moving rate "p". Also, "A", "B" and "C" are the quadratic, linear, and 0-order coefficients of the approximation formula, respectively.

In this case, it is also possible to consider a configuration in which the pulse rate estimation unit 130 performs fitting of a quadratic curve with the data of each subject held in the user-specific pulse estimation table 780, thus calculates the values of the coefficients "A" to "C" in the equation (1), calculates the pulse rate by applying the moving rate calculated by the moving rate calculation unit 120 to the equation (1) with the coefficients "A" to "C" decided, and defines the calculated pulse rate as the estimated pulse rate 755.

Also, the calories burned may be calculated from the estimated pulse rate 755 and information about the calories burned thus calculated may be displayed on the display unit 300. Moreover, the pulse rate and calories burned of the subject are not limited to being displayed on the display unit 300. It is also possible to consider a printing device, a projection device and a communication device or the like as the output unit, and outputting the pulse rate and calories burned to these devices.

Also, while the wristwatch-type pulsimeter 1 is considered as the biological information processing device, the device may also be applied to a finger-mounted pulsimeter which is mounted on a fingertip and measures pulses. Moreover, the method for detecting pulse wave signals is not limited to the detection method using light and may be a detection method using ultrasonic waves or infrared rays.

Also, the body motion sensor 20 is not limited to the configuration including an acceleration sensor. A configuration in which another sensor such as a velocity sensor detects body motions can also be considered. Moreover, the body motion sensor 20 may have both an acceleration sensor and a velocity sensor and detect body motions of the subject on the basis of signals outputted from the two sensors.

Also, if the user uses the device in a special environment such as on a high mountain, it is also possible to consider a configuration in which the pulse rate estimation unit 130 uses a pulse estimation table corresponding to the special environment that is prepared in advance, as the table to refer to, instead of the user-specific pulse estimation table 780. In this case, it is preferable that the pulse rate estimation unit 130 verifies the continuity of the two tables and executes transitions between the tables at such timings that the continuity will not be impaired.

The invention is not limited to the above embodiments and various changes and improvements can be added. Modifications will be described below.

(Modification 1)

In the above embodiments, the range of the window width 742 as a predetermined range based on the estimated pulse rate 755 is set to ±10 beats, and the SN threshold 746 is calculated on the basis of whether the measured pulse rate is within the range of the window width or not. In addition to this example, the value of the SN threshold 746 may be changed in multiple stages according to the degree of discrepancy between the estimated pulse rate 755 and the measured pulse rate. More specifically, if the discrepancy is small, the SN threshold 746 is set to a small value to loosen the SN determination criterion, and if the discrepancy is large, the SN threshold is set to a large value to tighten the SN determination criterion. Divisions of approximately four stages are provided according to the state of discrepancy, and four corresponding thresholds are set as the SN threshold 746. In this way, whether the measured pulse rate is appropriate or not can be determined more precisely, by performing the determination in multiple stages. A preferred example of setting the SN threshold 746 in multiple stages according to the degree of discrepancy is disclosed in JP-A-2013-13644.

(Modification 2)

In the above embodiments and modification, it is assumed that the window processing determination unit 136 performs determination on the basis of the estimated pulse rate 755. However, the reference value is not limited to the estimated pulse rate, and whether the measured pulse rate is appropriate or not may also be determined, using a maximum pulse rate and a minimum pulse rate. More specifically, the maximum pulse rate and the minimum pulse rate are acquired on the basis of the decided pulse rate 738, which is the accumulated data of the subject stored in the storage unit 700. If the measured pulse rate that is calculated is a value above the maximum pulse rate, the measured pulse rate may have been calculated inappropriately and therefore the SN determination criterion is tightened. If the measured pulse rate that is calculated is a value below the minimum pulse rate (if the subject is exercising as indicated by the body motion information of the subject, the value 1.2 times the minimum pulse rate, or the like), the SN determination criterion is tightened. In this way, as the SN determination unit 144 determines the measured pulse rate departing from the range between the maximum pulse rate and the minimum pulse rate, using a stricter SN determination criterion, an appropriate measured pulse rate can be decided as the pulse rate of the subject.

(Modification 3)

In the above embodiments and modifications, the estimated pulse rate calculation unit 132 finds the target pulse rate, using the moving rate. However, the target pulse rate and the estimated pulse rate may be found, using action patterns. More specifically, in circumstances where the body motion information (moving rate or the like) of the subject does not easily appear, it is difficult to use the moving rate. The moving rate does not easily appear, for example, during the period until the subject actually starts exercising after the subject puts on the pulsimeter 1, presses an operation button 5 and starts measurement. During this period, the measured pulse rate is calculated if measurement is already started, even when the subject is in standby for exercise or is stretching or the like.

In such circumstances, information for discriminating action patterns of the subject is gathered and the measured pulse rate is stored for each action pattern. If the tendency of the pulse rate comes to appear repeatedly on the basis of the measured pulse rate for each action pattern that is accumulated, the pulse rate is stored in the action-specific target pulse rate table 752 as the estimated pulse rate 755. Subsequently, the estimated pulse rate 755 can be derived for each action pattern.

As a specific example, in the calculation of the estimated pulse rate 755 during the period from the start of measurement to the start of exercise as described above, information such as "no moving rate appears", "there is little accumulation of the amount of change in body motion, " and "the moving rate appears with the lapse of a predetermined time" is stored as the body motion information and discriminated as action patterns during the period from the start of measurement to the start of exercise. The measured pulse rates are measured in such action patterns, and if the tendency emerges (the pulse rates gather in a predetermined range), the pulse rates can be defined as the estimated pulse rate 755.

In this way, since the estimated pulse rate 755 is derived even if the moving rate does not appear in the body motion information, the discrimination can be made on the basis of the estimated pulse rate regardless of whether the subject is exercising or not.

Also, the device which carries out the technique as described above may be realized by a single device or may be realized by combining a plurality of devices. Various configurations can be included.

The respective configurations in each embodiment and the combinations thereof are examples, and addition, omission, and replacement of a configuration, and other alterations are possible without departing from the scope of the invention. Also, the invention is not limited by the embodiments but only limited by the claims.

Reference Signs List

1 ... pulsimeter, 2 ... wristband, 3 ... case, 4 ... liquid crystal display unit, 5 ... operation button, 6 ... recharging terminal, 10 ... pulse wave sensor, 11 ... mirror surface, 12 ... light source, 13 ... light receiving element, 14 ... substrate, 20 ... body motion sensor, 30 ... pulse wave signal amplifying circuit unit, 40 ... pulse waveform shaping circuit unit, 50 ... body motion signal amplifying circuit unit, 60 ... body motion waveform shaping circuit unit, 70 ... A/D conversion unit, 100 ... processing unit, 110 ... pulse rate measurement unit, 120 ... moving rate calculation unit, 130 ... pulse rate estimation unit, 140 ... display control unit, 148 ... display control unit, 150 ... stability determination unit, 155 ... signal state acquisition unit, 160 ... reliability determination unit, 170 ... table update unit, 180 ... user setting unit, 200 ... operation unit, 300 ... display unit, 400 ... notification unit, 500 ... communication unit, 600 ... clock unit, 700 ... storage unit, 710 ... main program, 720 ... first reliability determination program, 722 ... action analysis program, 724 ... reliability determination program, 730 ... second reliability determination program, 732 ... pulse rate calculation program, 734 ... measured pulse rate, 736 ... moving rate, 738 ... decided pulse rate, 742 ... window width, 744 ... SN ratio, 746 ... SN threshold, 748 ... subject target pulse rate table, 750 ... measured pulse rate, 752 ... action-specific target pulse rate table, 755 ... estimated pulse rate, 760 ... moving rate stability, 765 ... pulse stability, 780 ... user-specific pulse estimation table.

## Claims

**1.** A biological information processing device comprising:

a biological information detection unit which detects biological information about a subject;
a body motion information detection unit which detects body motion information about a movement of the subject;
a reliability determination unit which determines reliability of the biological information on the basis of a predetermined criterion;
a storage unit which stores a correlation between the body motion information and the biological information; and
an update unit which updates the correlation stored in the storage unit;
wherein if the reliability determination unit determines that the biological information satisfies the predetermined criterion, the update unit updates the correlation on the basis of the biological information and the body motion information.

**2.** The biological information processing device according to claim 1, comprising:

an estimation unit which outputs estimated biological information from the body motion information detected by the body motion information detection unit, on the basis of the correlation stored in the storage unit;
a decision unit which decides one of the biological information and the estimated biological information as output information on the basis of a result of determination by the reliability determination unit; and
an output unit which outputs the output information decided by the decision unit.

3. The biological information processing device according to claim 2, wherein
the reliability determination unit determines the reliability of the biological information as one of a first determination, a second determination and a third determination, on the basis of the predetermined criterion,
the decision unit decides the estimated biological information estimated by the estimation unit, as the output information, if the first determination is made on the biological information, whereas the decision unit decides the biological information detected by the biological information detection unit, as the output information, if the second determination or the third determination is made on the biological information, and
the update unit updates the correlation if the third determination is made on the biological information.

4. The biological information processing device according to one of claims 1 to 3, wherein
the reliability determination unit determines the reliability of the biological information on the basis of at least one of stability of the biological information detected by the biological information detection unit and noise ratio information indicating the ratio of a noise component included in a signal indicating the biological information.

5. The biological information processing device according to one of claims 1 to 4, wherein
the update unit generates a third form of the correlation on the basis of a first form of the correlation stored in the storage unit at the start of detection of the biological information by the biological information detection unit and a second form of the correlation updated from the first form of the correlation at the time of detection of the biological information by the biological information detection unit, and stores the third form of the correlation thus generated, in the storage unit.

6. The biological information processing device according to claim 5, wherein
the update unit stores the third form of the correlation in the storage unit if the difference between the first form of the correlation and the second form of the correlation is within a predetermined range.

7. The biological information processing device according to one of claims 1 to 6, wherein
the update unit does not update the correlation regardless of the result of determination by the reliability determination unit, if the relation between the biological information and the body motion information is greatly different from the correlation.

8. A biological information processing method comprising:

a biological information detection process of detecting biological information about a subject;
a body motion information detection process of detecting body motion information about a movement of the subject;
a reliability determination process of determining reliability of the biological information on the basis of a predetermined criterion;
a storage process of storing a correlation between the body motion information and the biological information; and
an update process of updating the correlation on the basis of the biological information and the body motion information if the biological information is determined as satisfying the predetermined criterion in the reliability determination process.

9. A biological information processing device comprising:

a biological information detection unit which detects biological information about a subject;
a body motion information detection unit which detects body motion information about a movement of the subject;
an activity information derivation unit which derives activity information of the subject from the body motion information;
a storage unit which stores a correlation between the body motion information and the biological information in association with the activity information;
an estimation unit which acquires, from the storage unit, the correlation corresponding to the activity information derived by the activity information derivation unit, and decides estimated biological information from the body motion information detected by the body motion information detection unit on the basis of the correlation thus acquired;
a reliability determination unit which determines reliability of the biological information; and

a decision unit which decides one of the biological information and the estimated biological information as output information on the basis of a result of determination by the reliability determination unit.

**10.** The biological information processing device according to claim 9, wherein the activity information derivation unit derives the activity information of the subject on the basis of periodicity of an acceleration signal acquired from the body motion information.

**11.** The biological information processing device according to claim 10, wherein if the activity information derivation unit determines that the acceleration signal has the periodicity, the activity information derivation unit calculates an average pace during a predetermined period of time on the basis of a number of vibrations corresponding to the periodicity and determines whether the activity of the subject is walking or running, according to the value of the average pace.

**12.** The biological information processing device according to claim 11, wherein if the activity information derivation unit determines that the activity of the subject is walking, the correlation corresponding to walking is acquired from the storage unit, whereas if the activity information derivation unit determines that the activity of the subject is running, the correlation corresponding to running is acquired from the storage unit.

**13.** The biological information processing device according to claim 10, wherein if the activity information derivation unit determines that the acceleration signal does not have the periodicity, the activity information derivation unit analyzes an intensity of the acceleration signal and determines that the activity of the subject is a non-periodical exercise which causes a rise in pulse rate, on the basis of a number of occurrences of a predetermined intensity.

**14.** The biological information processing device according to claim 13, wherein if the activity information derivation unit determines that the activity of the subject is the non-periodical exercise which causes the rise in pulse rate, the correlation corresponding to the non-periodical exercise which causes the rise in pulse rate is acquired from the storage unit.

**15.** The biological information processing device according to one of claims 9 to 14, wherein the decision unit decides the biological information as the output information if the reliability determination unit determines that the biological information satisfies a predetermined condition on reliability, and the decision unit decides the estimated biological information as the output information if the reliability determination unit determines that the biological information does not satisfy the predetermined condition.

**16.** A biological information processing device comprising:

a detection unit which detects a measured pulse rate on the basis of biological information of a subject;
an estimation unit which estimates an estimated pulse rate on the basis of body motion information of the subject;
a first determination unit which determines whether the measured pulse rate is included in a predetermined range prescribed on the basis of the estimated pulse rate, or not;
a reliability degree information calculation unit which calculates reliability degree information on the basis of a frequency characteristic of the measured pulse rate;
a second determination unit which determines whether or not the reliability degree information exceeds a predetermined reference value prescribing whether the measured pulse rate has reliability or not; and
a decision unit which decides one of the measured pulse rate and the estimated pulse rate as a pulse rate of the subject on the basis of results of determination by the first determination unit and the second determination unit.

**17.** The biological information processing device according to claim 16, wherein the first determination unit sets an upper limit value and a lower limit value on the basis of the estimated pulse rate and uses a range prescribed by the upper limit value and the lower limit value, as the predetermined range.

**18.** The biological information processing device according to claim 16 or 17, wherein the estimation unit derives the estimated pulse rate from the body motion information, using a computational formula or correlation table that expresses a correlation between the body motion information and the estimated pulse rate.

**19.** The biological information processing device according to one of claims 16 to 18, further comprising

a reference value calculation unit which calculates the predetermined reference value for determining reliability of the measured pulse rate, on the basis of the result of determination by the first determination unit.

**20.** The biological information processing device according to one of claims 16 to 19, wherein
the second determination unit determines the reliability of the measured pulse rate, using the predetermined reference value, and
the decision unit decides the measured pulse rate as the pulse rate of the subject if the measured pulse rate has reliability, and decides the estimated pulse rate as the pulse rate of the subject if the measured pulse rate does not have reliability.

**Amended claims under Art. 19.1 PCT**

**1.** A biological information processing device comprising:

a biological information detection unit which detects biological information about a subject;
a body motion information detection unit which detects body motion information about a movement of the subject;
a reliability determination unit which determines reliability of the biological information on the basis of a predetermined criterion;
a storage unit which stores a correlation between the body motion information and the biological information; and
an update unit which updates the correlation stored in the storage unit;
wherein if the reliability determination unit determines that the biological information satisfies the predetermined criterion, the update unit updates the correlation on the basis of the biological information and the body motion information.

**2.** The biological information processing device according to claim 1, comprising:

an estimation unit which outputs estimated biological information from the body motion information detected by the body motion information detection unit, on the basis of the correlation stored in the storage unit;
a decision unit which decides one of the biological information and the estimated biological information as output information on the basis of a result of determination by the reliability determination unit; and
an output unit which outputs the output information decided by the decision unit.

**3.** The biological information processing device according to claim 2, wherein
the reliability determination unit determines the reliability of the biological information as one of a first determination, a second determination and a third determination, on the basis of the predetermined criterion,
the decision unit decides the estimated biological information estimated by the estimation unit, as the output information, if the first determination is made on the biological information, whereas the decision unit decides the biological information detected by the biological information detection unit, as the output information, if the second determination or the third determination is made on the biological information, and
the update unit updates the correlation if the third determination is made on the biological information.

**4.** The biological information processing device according to one of claims 1 to 3, wherein
the reliability determination unit determines the reliability of the biological information on the basis of at least one of stability of the biological information detected by the biological information detection unit and noise ratio information indicating the ratio of a noise component included in a signal indicating the biological information.

**5.** The biological information processing device according to one of claims 1 to 4, wherein
the update unit generates a third form of the correlation on the basis of a first form of the correlation stored in the storage unit at the start of detection of the biological information by the biological information detection unit and a second form of the correlation updated from the first form of the correlation at the time of detection of the biological information by the biological information detection unit, and stores the third form of the correlation thus generated, in the storage unit.

**6.** The biological information processing device according to claim 5, wherein
the update unit stores the third form of the correlation in the storage unit if the difference between the first form of the correlation and the second form of the correlation is within a predetermined range.

7. The biological information processing device according to one of claims 1 to 6, wherein the update unit does not update the correlation regardless of the result of determination by the reliability determination unit, if the relation between the biological information and the body motion information is greatly different from the correlation.

8. A biological information processing method comprising:

a biological information detection process of detecting biological information about a subject;
a body motion information detection process of detecting body motion information about a movement of the subject;
a reliability determination process of determining reliability of the biological information on the basis of a predetermined criterion;
a storage process of storing a correlation between the body motion information and the biological information; and
an update process of updating the correlation on the basis of the biological information and the body motion information if the biological information is determined as satisfying the predetermined criterion in the reliability determination process.

9. A biological information processing device comprising:

a biological information detection unit which detects biological information about a subject;
a body motion information detection unit which detects body motion information about a movement of the subject;
an activity information derivation unit which derives activity information of the subject from the body motion information;
a storage unit which stores a correlation between the body motion information and the biological information in association with the activity information;
an estimation unit which acquires, from the storage unit, the correlation corresponding to the activity information derived by the activity information derivation unit, and decides estimated biological information from the body motion information detected by the body motion information detection unit on the basis of the correlation thus acquired;
a reliability determination unit which determines reliability of the biological information; and
a decision unit which decides one of the biological information and the estimated biological information as output information on the basis of a result of determination by the reliability determination unit;
wherein the activity information derivation unit derives the activity information of the subject on the basis of periodicity of an acceleration signal acquired from the body motion information, and if the activity information derivation unit determines that the acceleration signal has the periodicity, the activity information derivation unit calculates an average pace during a predetermined period of time on the basis of a number of vibrations corresponding to the periodicity and determines whether the activity of the subject is walking or running, according to the value of the average pace.

10. The biological information processing device according to claim 9, wherein if the activity information derivation unit determines that the activity of the subject is walking, the correlation corresponding to walking is acquired from the storage unit, whereas if the activity information derivation unit determines that the activity of the subject is running, the correlation corresponding to running is acquired from the storage unit.

11. The biological information processing device according to claim 9, wherein if the activity information derivation unit determines that the acceleration signal does not have the periodicity, the activity information derivation unit analyzes an intensity of the acceleration signal and determines that the activity of the subject is a non-periodical exercise which causes a rise in pulse rate, on the basis of a number of occurrences of a predetermined intensity.

12. The biological information processing device according to claim 11, wherein if the activity information derivation unit determines that the activity of the subject is the non-periodical exercise which causes the rise in pulse rate, the correlation corresponding to the non-periodical exercise which causes the rise in pulse rate is acquired from the storage unit.

13. The biological information processing device according to claim 9, wherein the decision unit decides the biological information as the output information if the reliability determination unit determines that the biological information satisfies a predetermined condition on reliability, and the decision unit decides the estimated biological information as the output information if the reliability determination unit determines that the biological information does not satisfy the predetermined condition.

**14.** A biological information processing device comprising:

a detection unit which detects a measured pulse rate on the basis of biological information of a subject;
an estimation unit which estimates an estimated pulse rate on the basis of body motion information of the subject;
a first determination unit which determines whether the measured pulse rate is included in a predetermined range prescribed on the basis of the estimated pulse rate, or not;
a reliability degree information calculation unit which calculates reliability degree information on the basis of a frequency characteristic of the measured pulse rate;
a second determination unit which determines whether or not the reliability degree information exceeds a predetermined reference value prescribing whether the measured pulse rate has reliability or not; and
a decision unit which decides one of the measured pulse rate and the estimated pulse rate as a pulse rate of the subject on the basis of results of determination by the first determination unit and the second determination unit.

**15.** The biological information processing device according to claim 14, wherein
the first determination unit sets an upper limit value and a lower limit value on the basis of the estimated pulse rate and uses a range prescribed by the upper limit value and the lower limit value, as the predetermined range.

**16.** The biological information processing device according to claim 14 or 15, wherein
the estimation unit derives the estimated pulse rate from the body motion information, using a computational formula or correlation table that expresses a correlation between the body motion information and the estimated pulse rate.

**17.** The biological information processing device according to one of claims 14 to 16, further comprising
a reference value calculation unit which calculates the predetermined reference value for determining reliability of the measured pulse rate, on the basis of the result of determination by the first determination unit.

**18.** The biological information processing device according to one of claims 14 to 17, wherein
the second determination unit determines the reliability of the measured pulse rate, using the predetermined reference value, and
the decision unit decides the measured pulse rate as the pulse rate of the subject if the measured pulse rate has reliability, and decides the estimated pulse rate as the pulse rate of the subject if the measured pulse rate does not have reliability.

The amended claim 9 is amended on the basis of the descriptions of the original claims 9, 10 and 11.

The amended claim 10 is equivalent to the original claim 12.

The amended claim 11 is equivalent to the original claim 13.

The amended claim 12 is equivalent to the original claim 14.

The amended claim 13 is equivalent to the original claim 15.

The amended claim 14 is equivalent to the original claim 16.

The amended claim 15 is equivalent to the original claim 17.

The amended claim 16 is equivalent to the original claim 18.

The amended claim 17 is equivalent to the original claim 19.

The amended claim 18 is equivalent to the original claim 20.

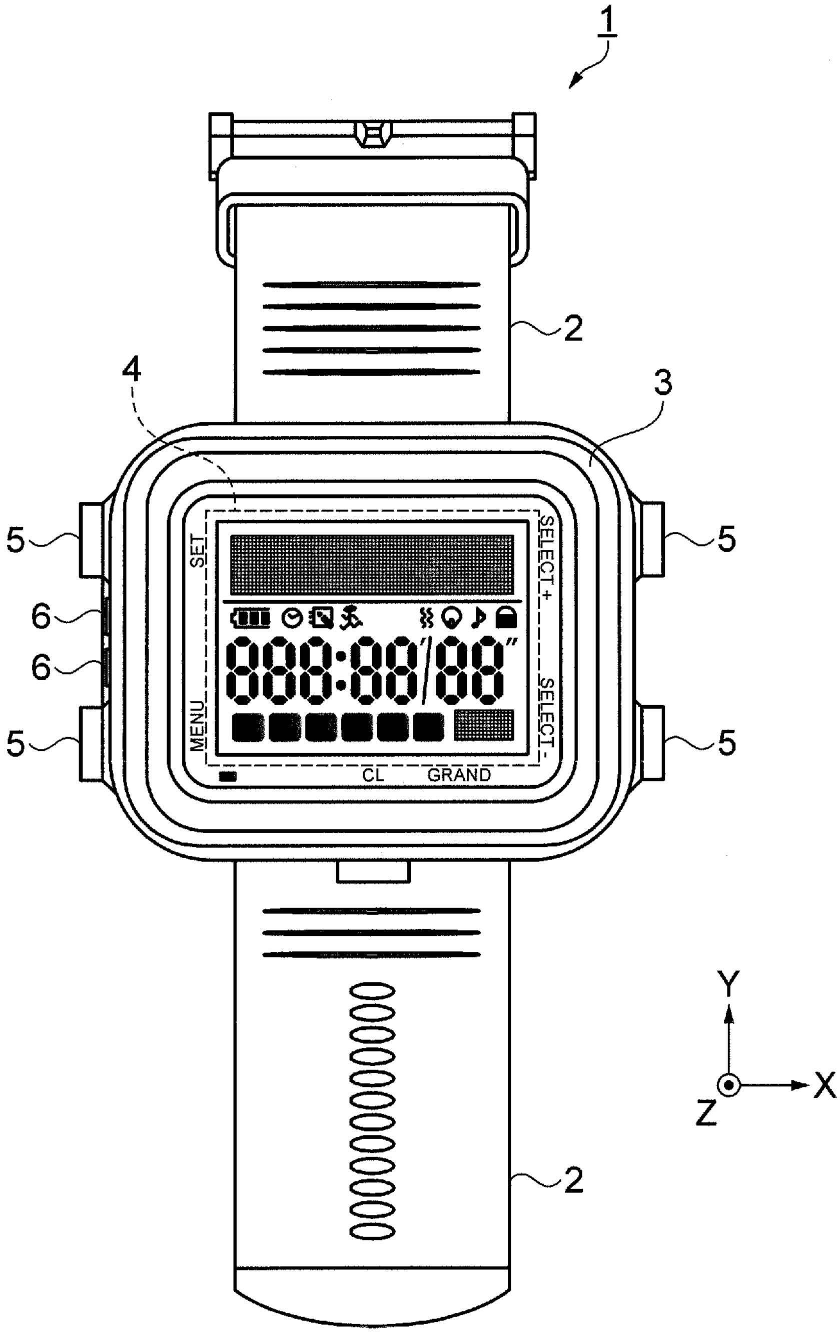


FIG. 1

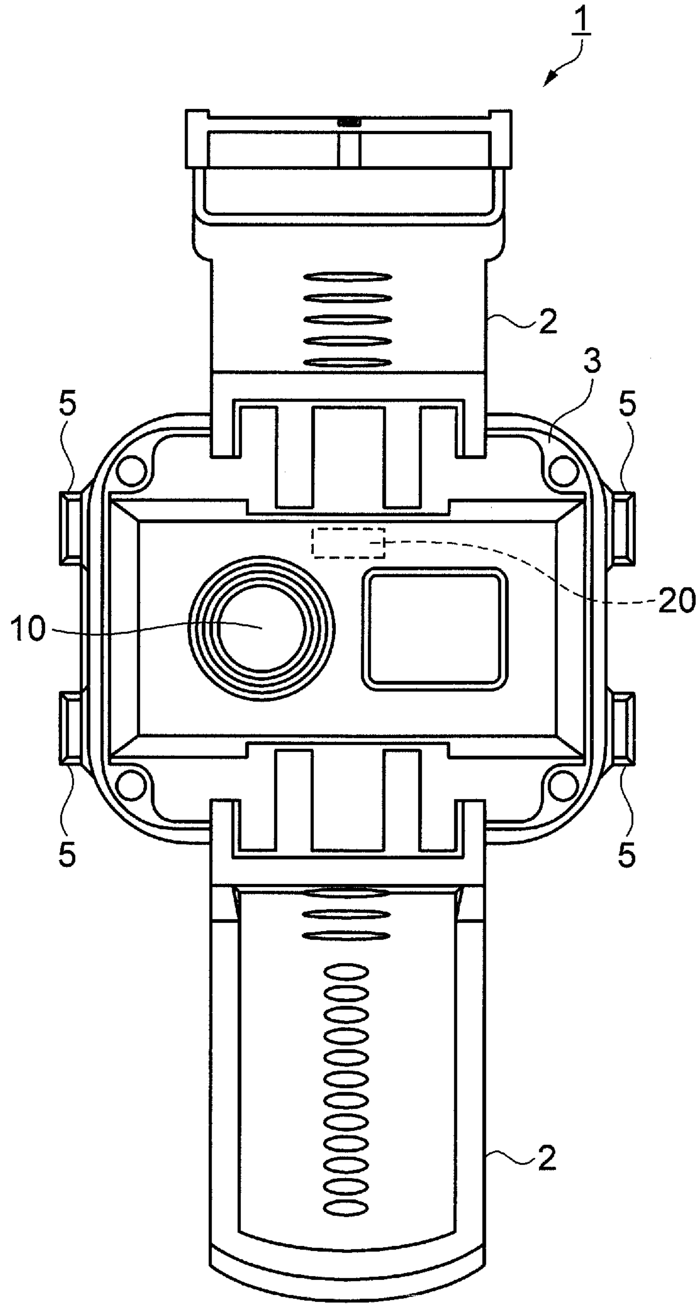


FIG. 2A

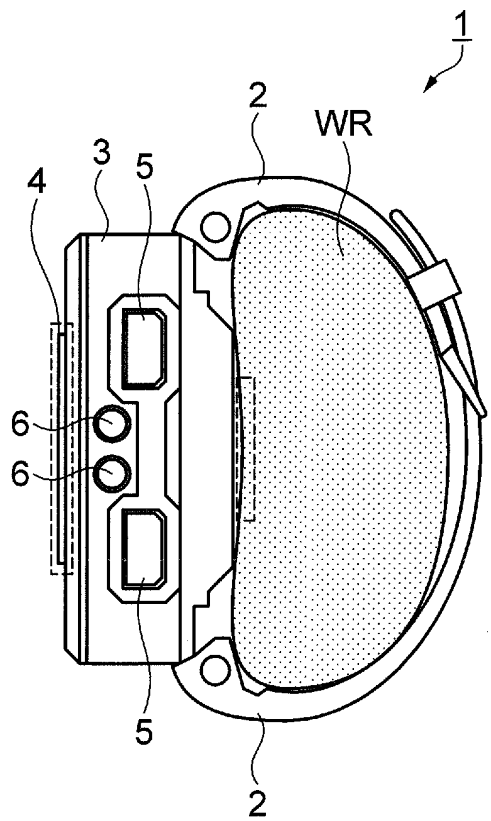


FIG. 2B

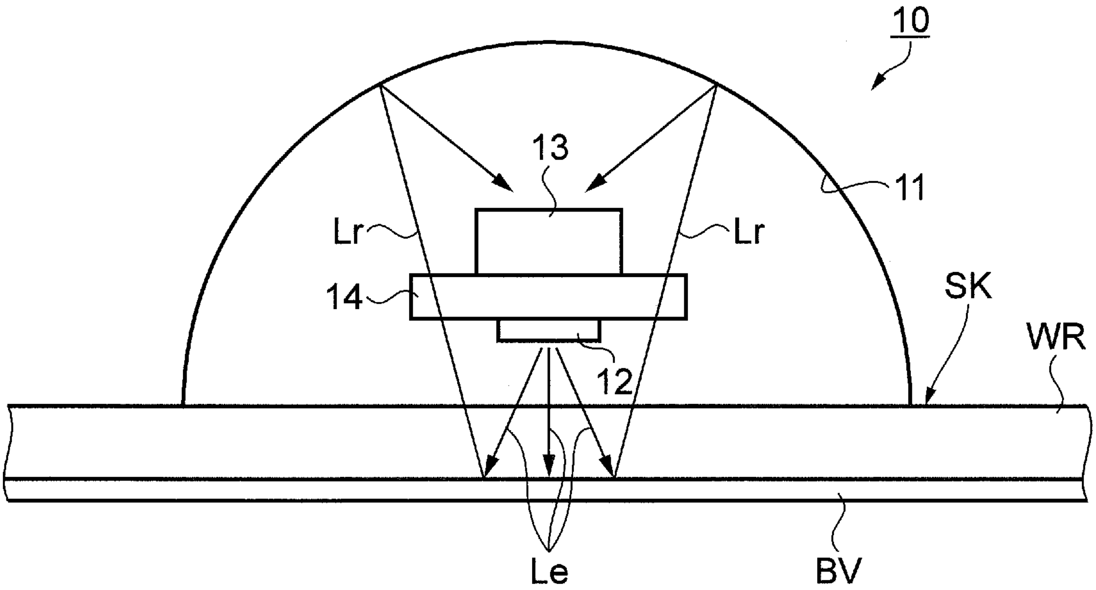


FIG. 3

FIG. 4

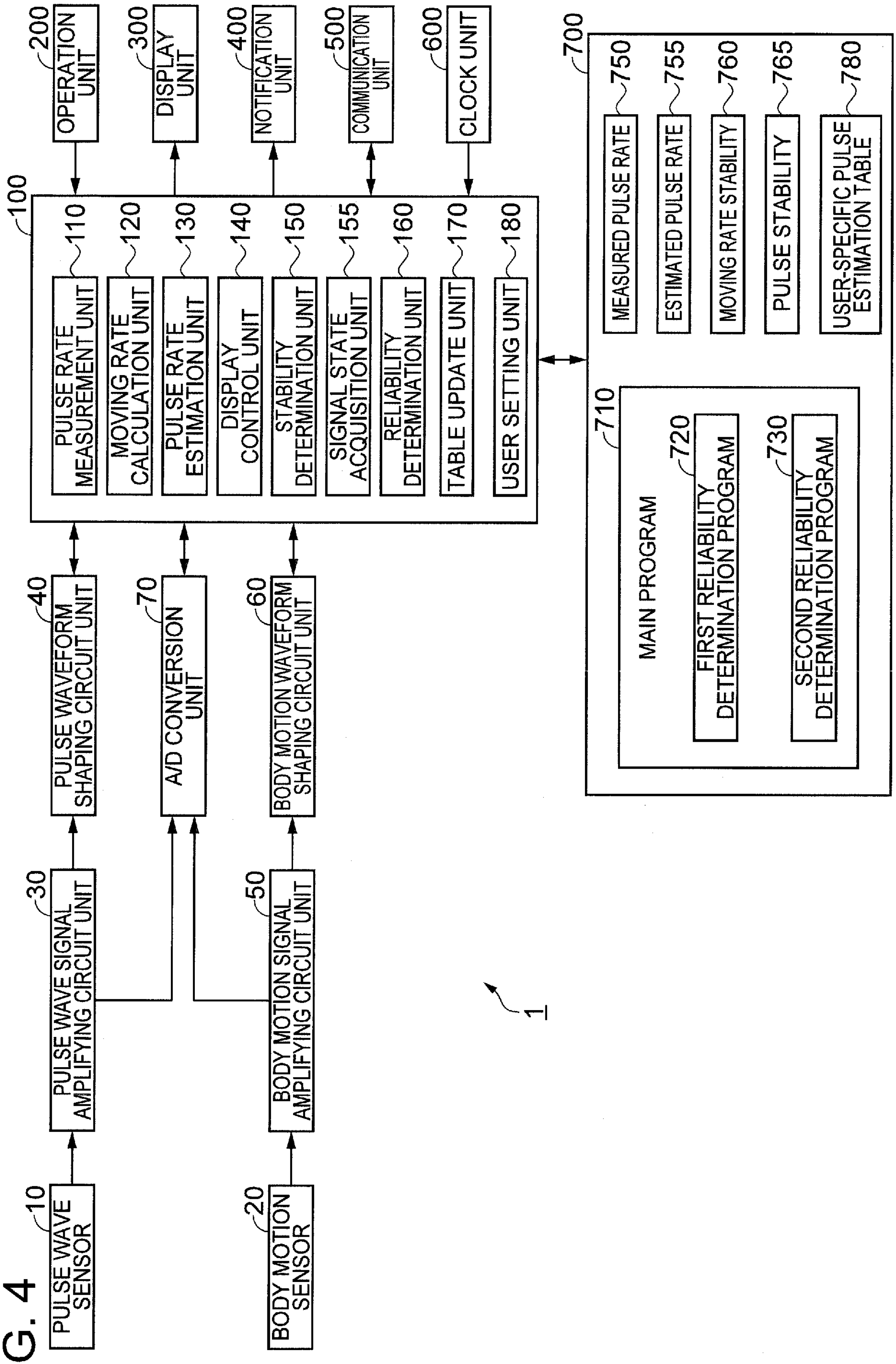
10 PULSE WAVE SENSOR
20 BODY MOTION SENSOR
30 PULSE WAVE SIGNAL AMPLIFYING CIRCUIT UNIT
50 BODY MOTION SIGNAL AMPLIFYING CIRCUIT UNIT
40 PULSE WAVEFORM SHAPING CIRCUIT UNIT
70 A/D CONVERSION UNIT
60 BODY MOTION WAVEFORM SHAPING CIRCUIT UNIT
100
110 PULSE RATE MEASUREMENT UNIT
120 MOVING RATE CALCULATION UNIT
130 PULSE RATE ESTIMATION UNIT
140 DISPLAY CONTROL UNIT
150 STABILITY DETERMINATION UNIT
155 SIGNAL STATE ACQUISITION UNIT
160 RELIABILITY DETERMINATION UNIT
170 TABLE UPDATE UNIT
180 USER SETTING UNIT
200 OPERATION UNIT
300 DISPLAY UNIT
400 NOTIFICATION UNIT
500 COMMUNICATION UNIT
600 CLOCK UNIT
700
710 MAIN PROGRAM
720 FIRST RELIABILITY DETERMINATION PROGRAM
730 SECOND RELIABILITY DETERMINATION PROGRAM
750 MEASURED PULSE RATE
755 ESTIMATED PULSE RATE
760 MOVING RATE STABILITY
765 PULSE STABILITY
780 USER-SPECIFIC PULSE ESTIMATION TABLE
1

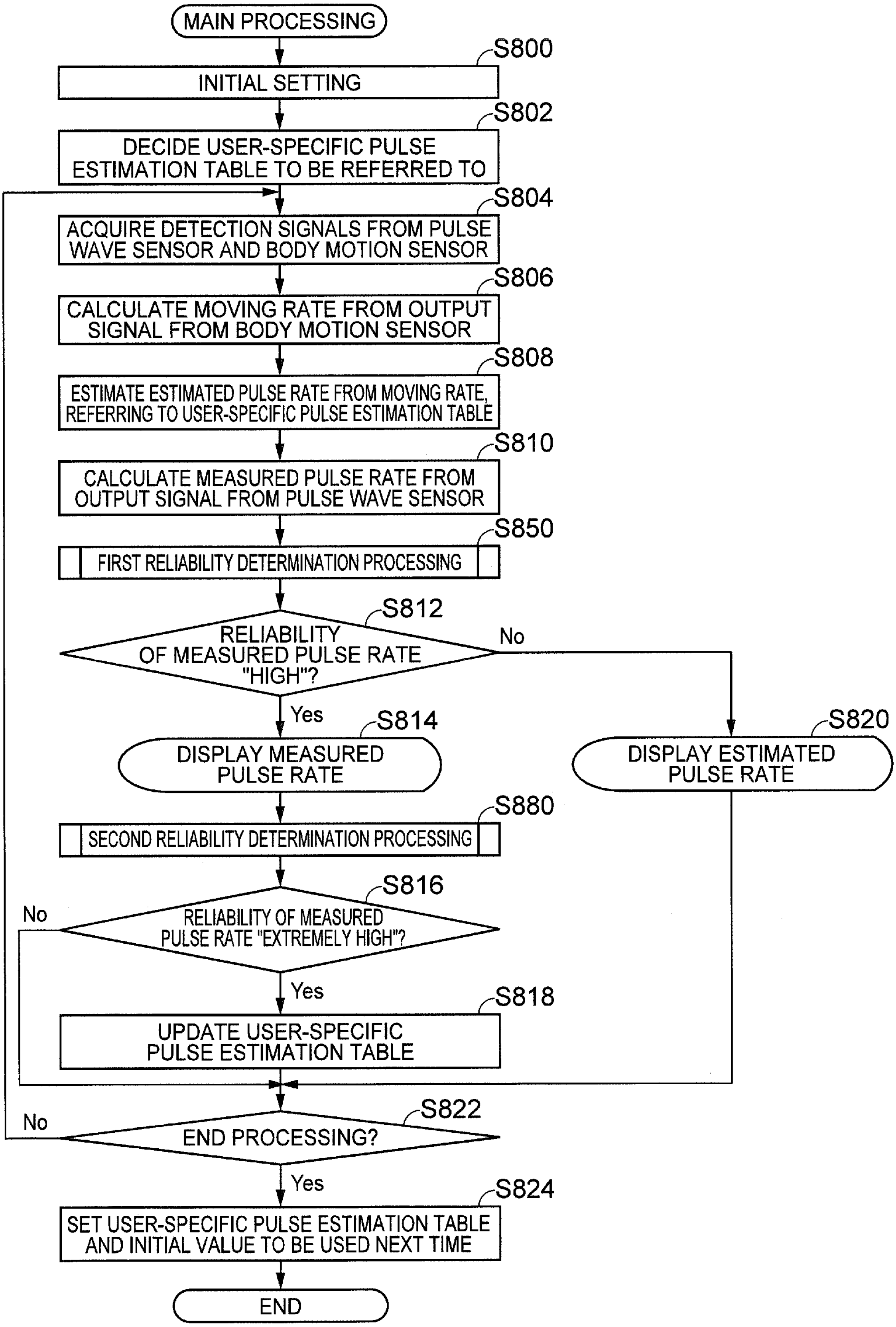
MAIN PROCESSING
S800
INITIAL SETTING
S802
DECIDE USER-SPECIFIC PULSE ESTIMATION TABLE TO BE REFERRED TO
S804
ACQUIRE DETECTION SIGNALS FROM PULSE WAVE SENSOR AND BODY MOTION SENSOR
S806
CALCULATE MOVING RATE FROM OUTPUT SIGNAL FROM BODY MOTION SENSOR
S808
ESTIMATE ESTIMATED PULSE RATE FROM MOVING RATE, REFERRING TO USER-SPECIFIC PULSE ESTIMATION TABLE
S810
CALCULATE MEASURED PULSE RATE FROM OUTPUT SIGNAL FROM PULSE WAVE SENSOR
S850
FIRST RELIABILITY DETERMINATION PROCESSING
S812
RELIABILITY OF MEASURED PULSE RATE "HIGH"?
No
Yes
S814
DISPLAY MEASURED PULSE RATE
S820
DISPLAY ESTIMATED PULSE RATE
S880
SECOND RELIABILITY DETERMINATION PROCESSING
S816
RELIABILITY OF MEASURED PULSE RATE "EXTREMELY HIGH"?
No
Yes
S818
UPDATE USER-SPECIFIC PULSE ESTIMATION TABLE
S822
No
END PROCESSING?
Yes
S824
SET USER-SPECIFIC PULSE ESTIMATION TABLE AND INITIAL VALUE TO BE USED NEXT TIME
END

FIG. 5

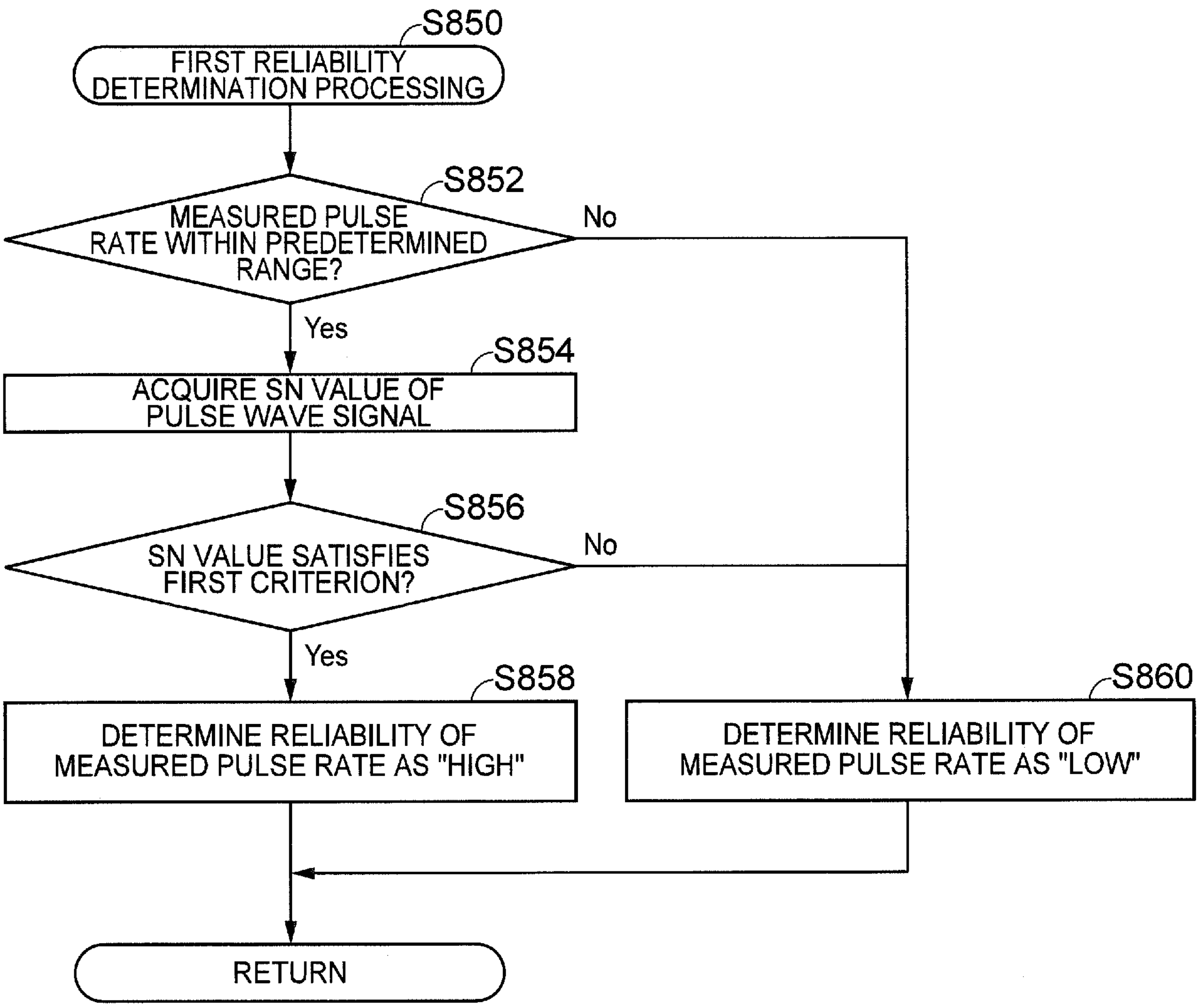
S850
FIRST RELIABILITY DETERMINATION PROCESSING
S852
MEASURED PULSE RATE WITHIN PREDETERMINED RANGE?
No
Yes
S854
ACQUIRE SN VALUE OF PULSE WAVE SIGNAL
S856
SN VALUE SATISFIES FIRST CRITERION?
No
Yes
S858
DETERMINE RELIABILITY OF MEASURED PULSE RATE AS "HIGH"
S860
DETERMINE RELIABILITY OF MEASURED PULSE RATE AS "LOW"
RETURN

FIG. 6

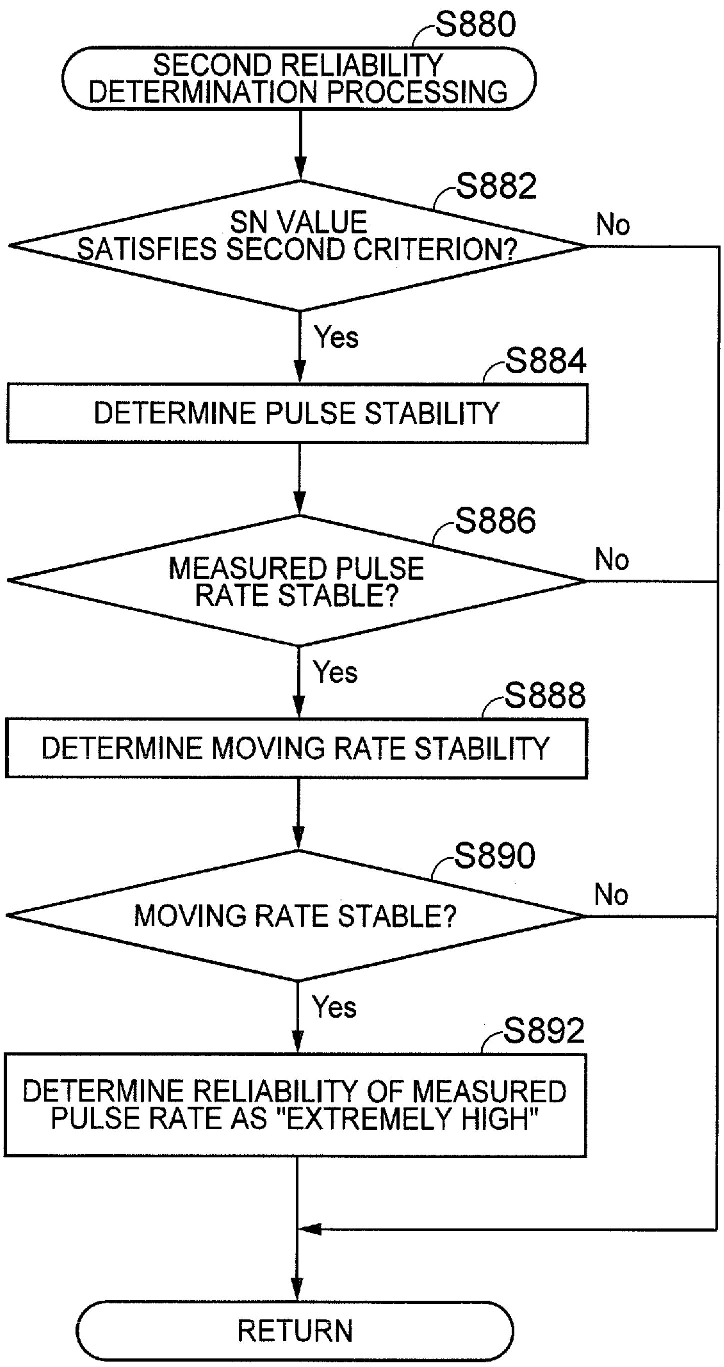
S880
SECOND RELIABILITY DETERMINATION PROCESSING
S882
SN VALUE SATISFIES SECOND CRITERION?
No
Yes
S884
DETERMINE PULSE STABILITY
S886
MEASURED PULSE RATE STABLE?
No
Yes
S888
DETERMINE MOVING RATE STABILITY
S890
MOVING RATE STABLE?
No
Yes
S892
DETERMINE RELIABILITY OF MEASURED PULSE RATE AS "EXTREMELY HIGH"
RETURN

FIG. 7

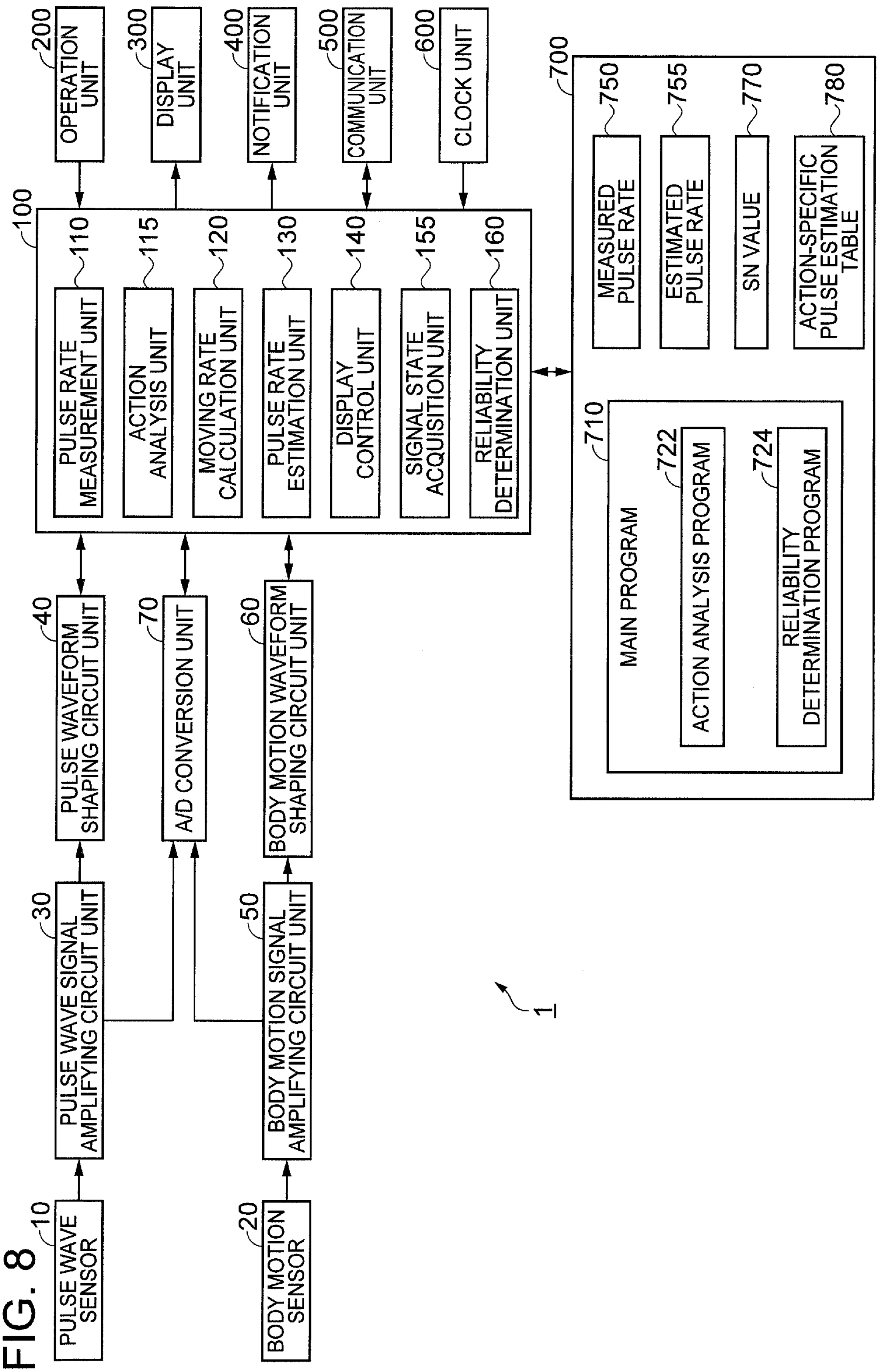
PULSE WAVE SENSOR
10
BODY MOTION SENSOR
20
PULSE WAVE SIGNAL AMPLIFYING CIRCUIT UNIT
30
BODY MOTION SIGNAL AMPLIFYING CIRCUIT UNIT
50
PULSE WAVEFORM SHAPING CIRCUIT UNIT
40
A/D CONVERSION UNIT
70
BODY MOTION WAVEFORM SHAPING CIRCUIT UNIT
60
100
PULSE RATE MEASUREMENT UNIT
110
ACTION ANALYSIS UNIT
115
MOVING RATE CALCULATION UNIT
120
PULSE RATE ESTIMATION UNIT
130
DISPLAY CONTROL UNIT
140
SIGNAL STATE ACQUISITION UNIT
155
RELIABILITY DETERMINATION UNIT
160
OPERATION UNIT
200
DISPLAY UNIT
300
NOTIFICATION UNIT
400
COMMUNICATION UNIT
500
CLOCK UNIT
600
700
MEASURED PULSE RATE
750
ESTIMATED PULSE RATE
755
SN VALUE
770
ACTION-SPECIFIC PULSE ESTIMATION TABLE
780
MAIN PROGRAM
710
ACTION ANALYSIS PROGRAM
722
RELIABILITY DETERMINATION PROGRAM
724
1

FIG. 8

FIG. 9

| MOVING RATE | PULSE RATE |
|---|---|
| ~ 91 | 92 |
| 92 ~ 97 | 92 |
| 98 ~ 103 | 93 |
| 104 ~ 109 | 94 |
| 110 ~ 115 | 96 |
| 116 ~ 121 | 100 |
| 122 ~ 127 | 104 |
| 128 ~ 133 | 109 |
| 134 ~ 139 | 116 |

PULSE ESTIMATION TABLE(WALKING)

| MOVING RATE | PULSE RATE |
|---|---|
| 140 ~ 142 | 121 |
| 143 ~ 145 | 122 |
| 146 ~ 148 | 124 |
| 149 ~ 151 | 125 |
| 152 ~ 154 | 128 |
| 155 ~ 157 | 130 |
| 158 ~ 160 | 133 |
| 161 ~ 163 | 137 |
| 164 ~ 166 | 141 |
| 167 ~ 169 | 145 |
| 170 ~ 172 | 150 |
| 173 ~ 175 | 155 |
| 176 ~ 178 | 161 |
| 179 ~ 181 | 167 |
| 182 ~ 184 | 173 |
| 185 ~ 187 | 180 |
| 188 ~ 190 | 187 |
| 191 ~ | 195 |

PULSE ESTIMATION TABLE(WALKING)

| INTENSITY OF ACCELERATION | PULSE RATE |
|---|---|
| ~ 800 | 78 |
| 801 ~ 3000 | 90 |
| 3001 ~ 6000 | 102 |
| 6001 ~ 9000 | 114 |
| 9001 ~ 12000 | 125 |
| 12001 ~ 15000 | 131 |
| 15001 ~ 18000 | 137 |
| 18001 ~ 21000 | 143 |
| 21001 ~ 24000 | 149 |
| 24001 ~ | 161 |

PULSE ESTIMATION TABLE(CALISTHENICS)

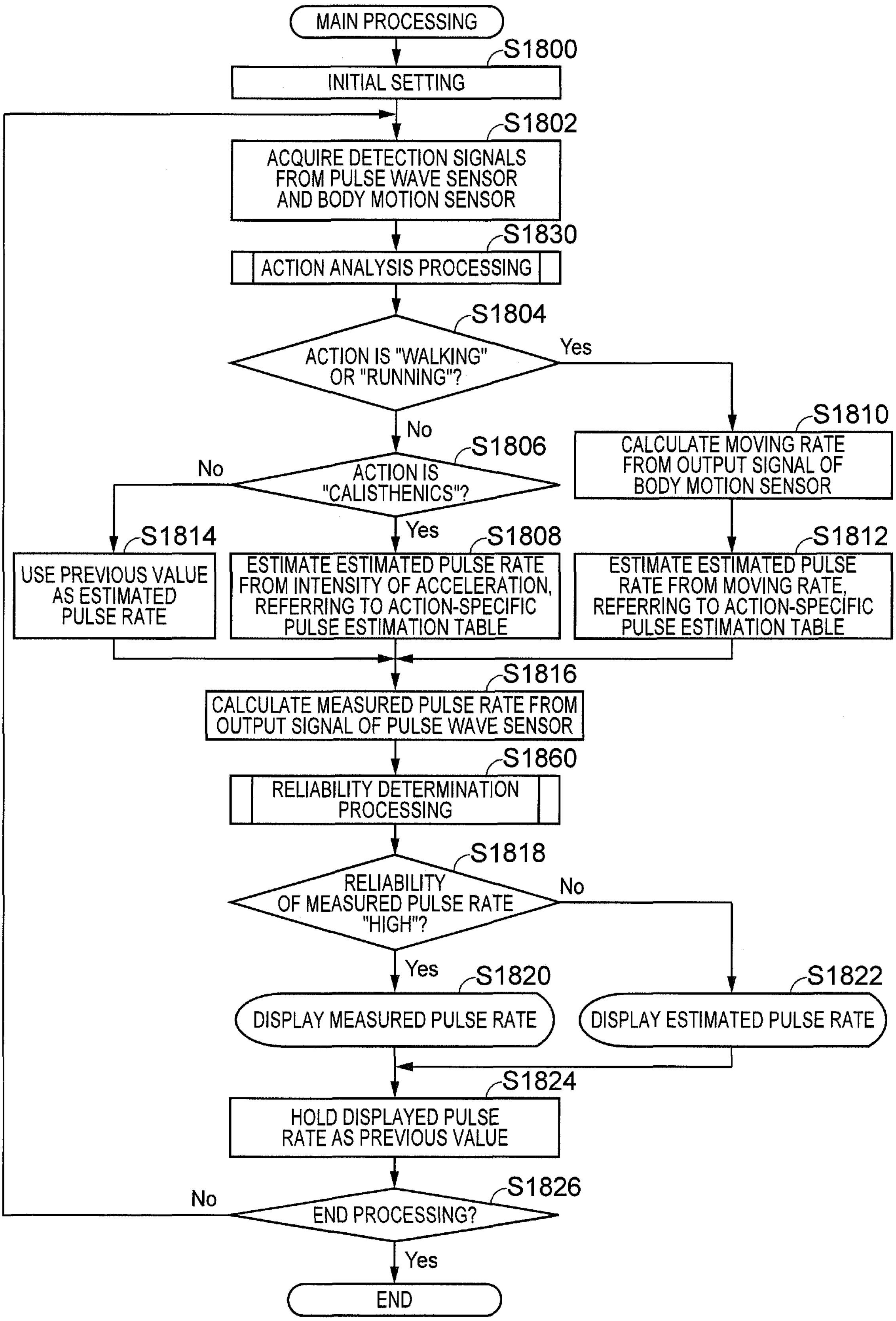
MAIN PROCESSING
S1800
INITIAL SETTING
S1802
ACQUIRE DETECTION SIGNALS FROM PULSE WAVE SENSOR AND BODY MOTION SENSOR
S1830
ACTION ANALYSIS PROCESSING
S1804
ACTION IS "WALKING" OR "RUNNING"?
Yes
No
S1810
CALCULATE MOVING RATE FROM OUTPUT SIGNAL OF BODY MOTION SENSOR
S1806
ACTION IS "CALISTHENICS"?
No
Yes
S1814
USE PREVIOUS VALUE AS ESTIMATED PULSE RATE
S1808
ESTIMATE ESTIMATED PULSE RATE FROM INTENSITY OF ACCELERATION, REFERRING TO ACTION-SPECIFIC PULSE ESTIMATION TABLE
S1812
ESTIMATE ESTIMATED PULSE RATE FROM MOVING RATE, REFERRING TO ACTION-SPECIFIC PULSE ESTIMATION TABLE
S1816
CALCULATE MEASURED PULSE RATE FROM OUTPUT SIGNAL OF PULSE WAVE SENSOR
S1860
RELIABILITY DETERMINATION PROCESSING
S1818
RELIABILITY OF MEASURED PULSE RATE "HIGH"?
No
Yes
S1820
DISPLAY MEASURED PULSE RATE
S1822
DISPLAY ESTIMATED PULSE RATE
S1824
HOLD DISPLAYED PULSE RATE AS PREVIOUS VALUE
S1826
END PROCESSING?
No
Yes
END

FIG. 10

FIG. 11

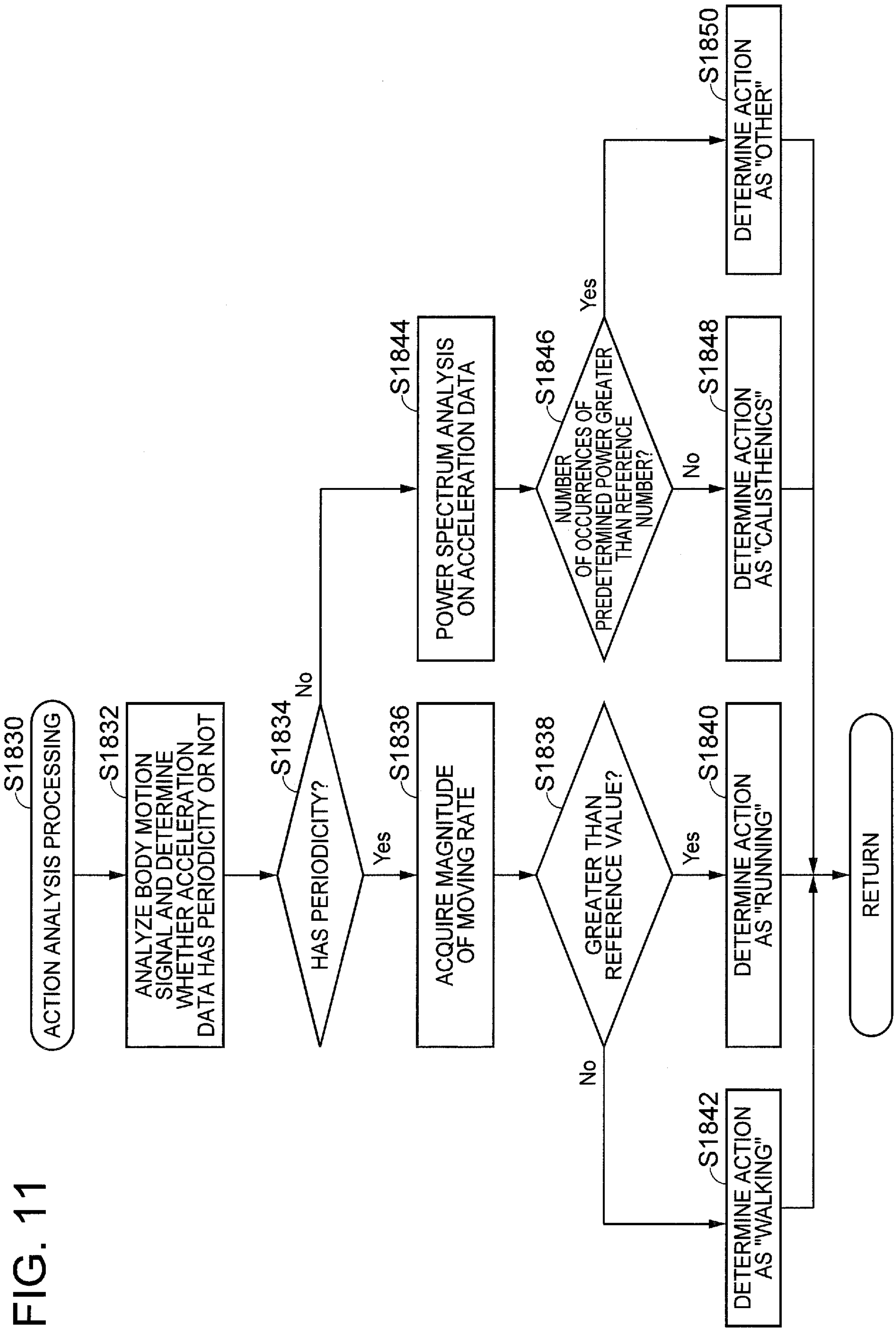
S1830
ACTION ANALYSIS PROCESSING
S1832
ANALYZE BODY MOTION SIGNAL AND DETERMINE WHETHER ACCELERATION DATA HAS PERIODICITY OR NOT
S1834
HAS PERIODICITY?
Yes
No
S1836
ACQUIRE MAGNITUDE OF MOVING RATE
S1838
GREATER THAN REFERENCE VALUE?
Yes
No
S1840
DETERMINE ACTION AS "RUNNING"
S1842
DETERMINE ACTION AS "WALKING"
S1844
POWER SPECTRUM ANALYSIS ON ACCELERATION DATA
S1846
NUMBER OF OCCURRENCES OF PREDETERMINED POWER GREATER THAN REFERENCE NUMBER?
Yes
No
S1848
DETERMINE ACTION AS "CALISTHENICS"
S1850
DETERMINE ACTION AS "OTHER"
RETURN

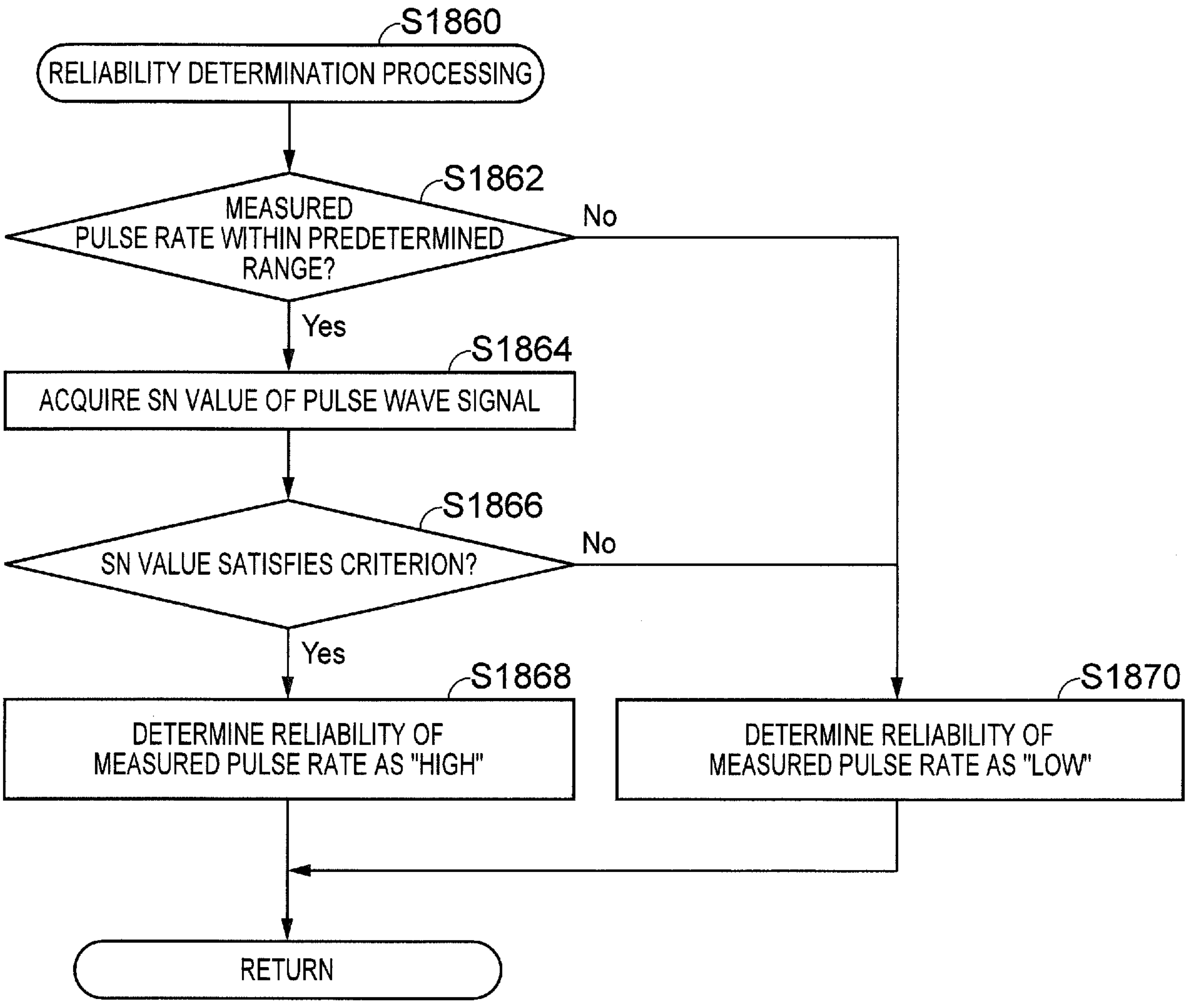
S1860
RELIABILITY DETERMINATION PROCESSING
S1862
MEASURED PULSE RATE WITHIN PREDETERMINED RANGE?
No
Yes
S1864
ACQUIRE SN VALUE OF PULSE WAVE SIGNAL
S1866
SN VALUE SATISFIES CRITERION?
No
Yes
S1868
DETERMINE RELIABILITY OF MEASURED PULSE RATE AS "HIGH"
S1870
DETERMINE RELIABILITY OF MEASURED PULSE RATE AS "LOW"
RETURN

FIG. 12

FIG. 13

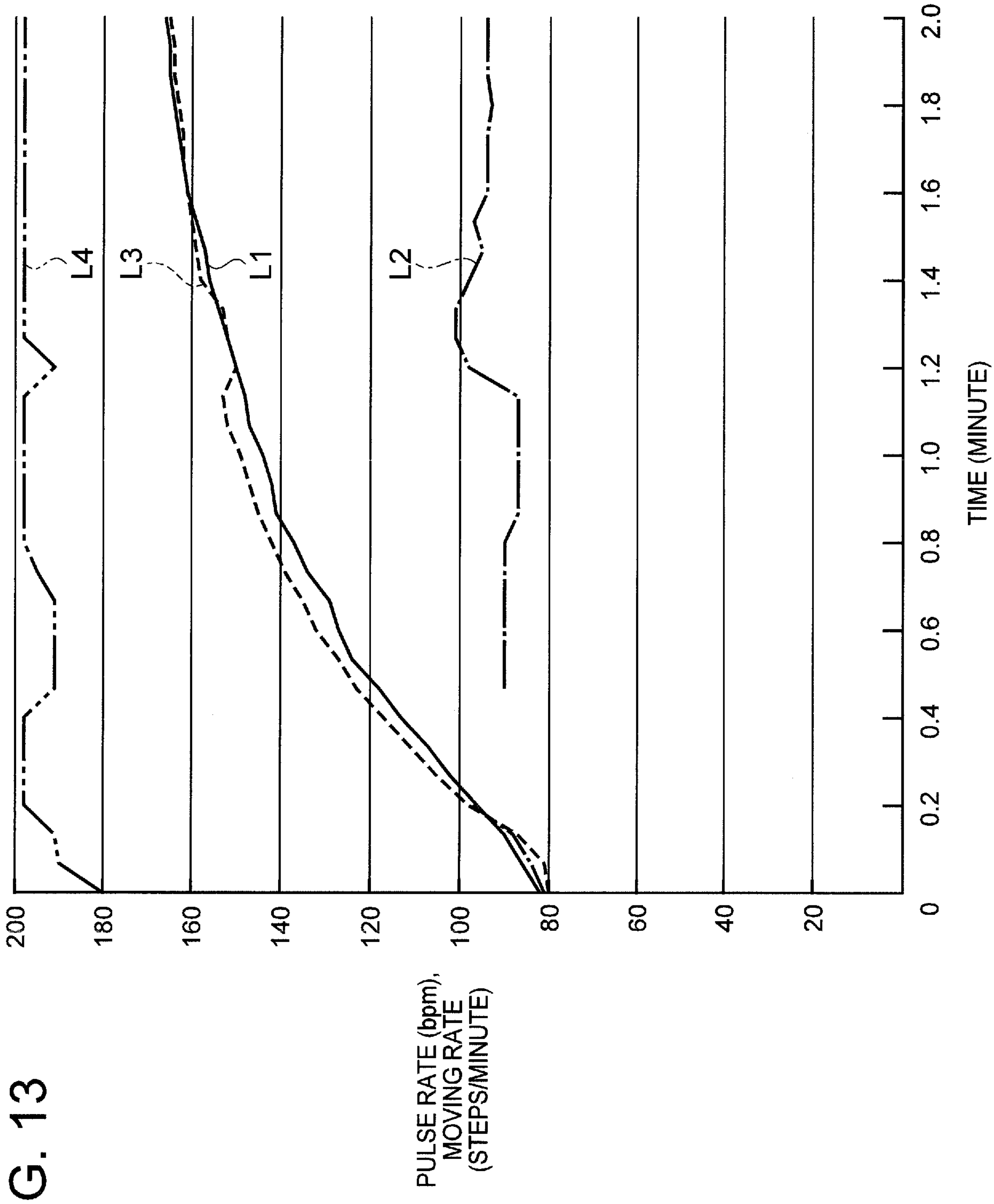
PULSE RATE (bpm), MOVING RATE (STEPS/MINUTE)
200
180
160
140
120
100
80
60
40
20
0
0.2
0.4
0.6
0.8
1.0
1.2
1.4
1.6
1.8
2.0
TIME (MINUTE)
L1
L2
L3
L4

FIG. 14

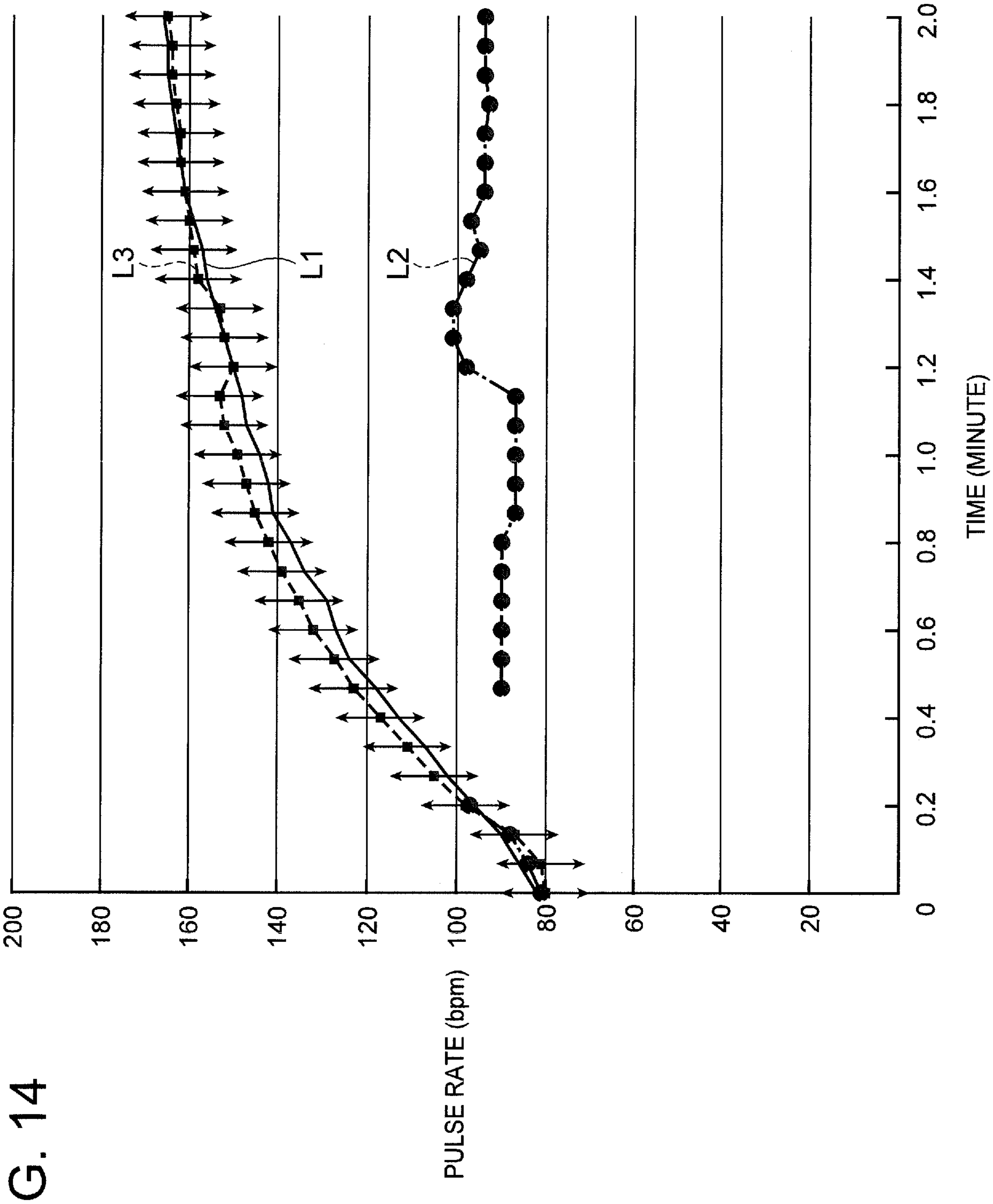
PULSE RATE (bpm)
200
180
160
140
120
100
80
60
40
20
0
0.2
0.4
0.6
0.8
1.0
1.2
1.4
1.6
1.8
2.0
TIME (MINUTE)
L1
L2
L3

FIG. 15

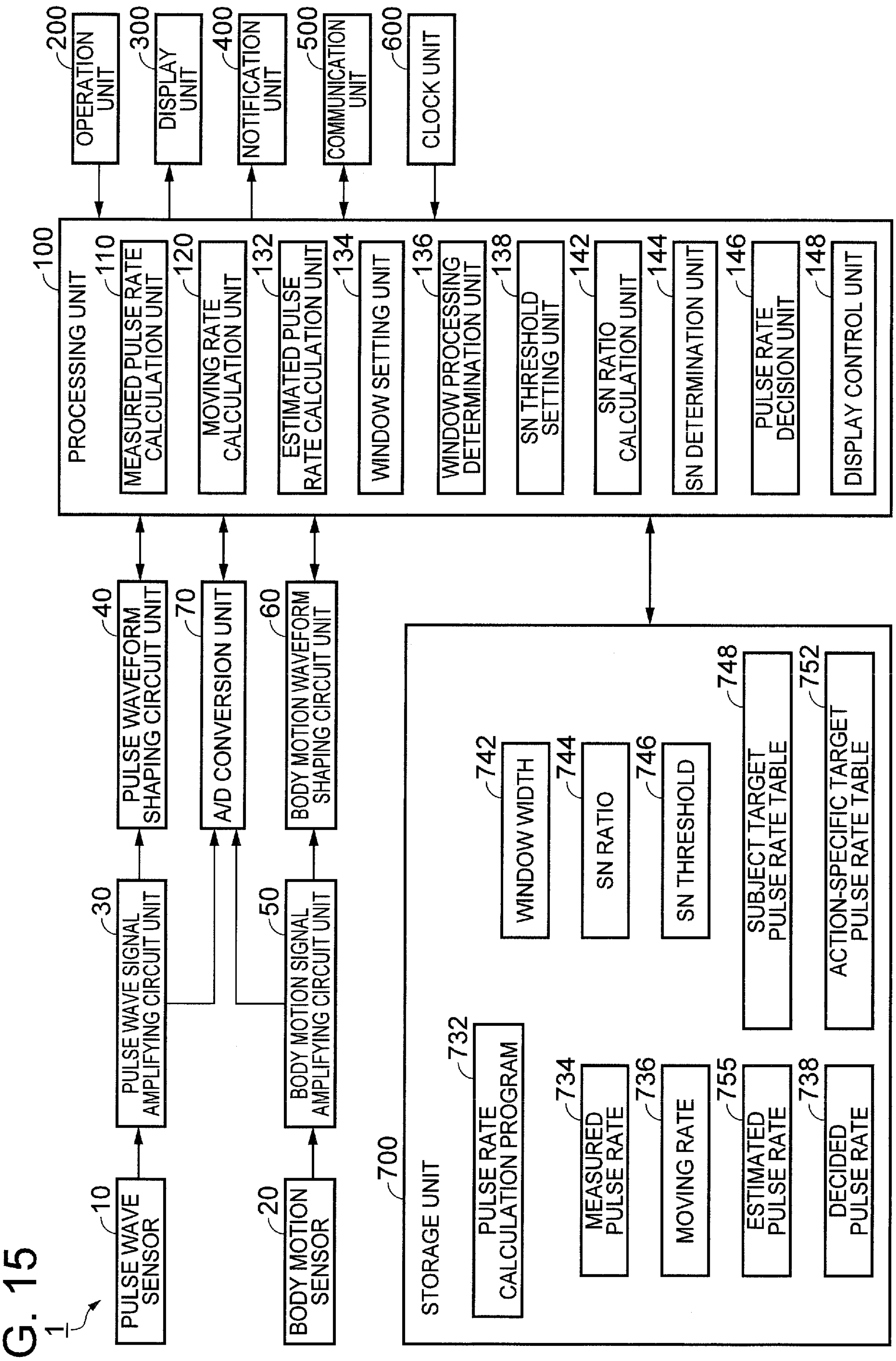
1
10 PULSE WAVE SENSOR
20 BODY MOTION SENSOR
30 PULSE WAVE SIGNAL AMPLIFYING CIRCUIT UNIT
50 BODY MOTION SIGNAL AMPLIFYING CIRCUIT UNIT
40 PULSE WAVEFORM SHAPING CIRCUIT UNIT
70 A/D CONVERSION UNIT
60 BODY MOTION WAVEFORM SHAPING CIRCUIT UNIT
100 PROCESSING UNIT
110 MEASURED PULSE RATE CALCULATION UNIT
120 MOVING RATE CALCULATION UNIT
132 ESTIMATED PULSE RATE CALCULATION UNIT
134 WINDOW SETTING UNIT
136 WINDOW PROCESSING DETERMINATION UNIT
138 SN THRESHOLD SETTING UNIT
142 SN RATIO CALCULATION UNIT
144 SN DETERMINATION UNIT
146 PULSE RATE DECISION UNIT
148 DISPLAY CONTROL UNIT
200 OPERATION UNIT
300 DISPLAY UNIT
400 NOTIFICATION UNIT
500 COMMUNICATION UNIT
600 CLOCK UNIT
700 STORAGE UNIT
732 PULSE RATE CALCULATION PROGRAM
734 MEASURED PULSE RATE
736 MOVING RATE
755 ESTIMATED PULSE RATE
738 DECIDED PULSE RATE
742 WINDOW WIDTH
744 SN RATIO
746 SN THRESHOLD
748 SUBJECT TARGET PULSE RATE TABLE
752 ACTION-SPECIFIC TARGET PULSE RATE TABLE

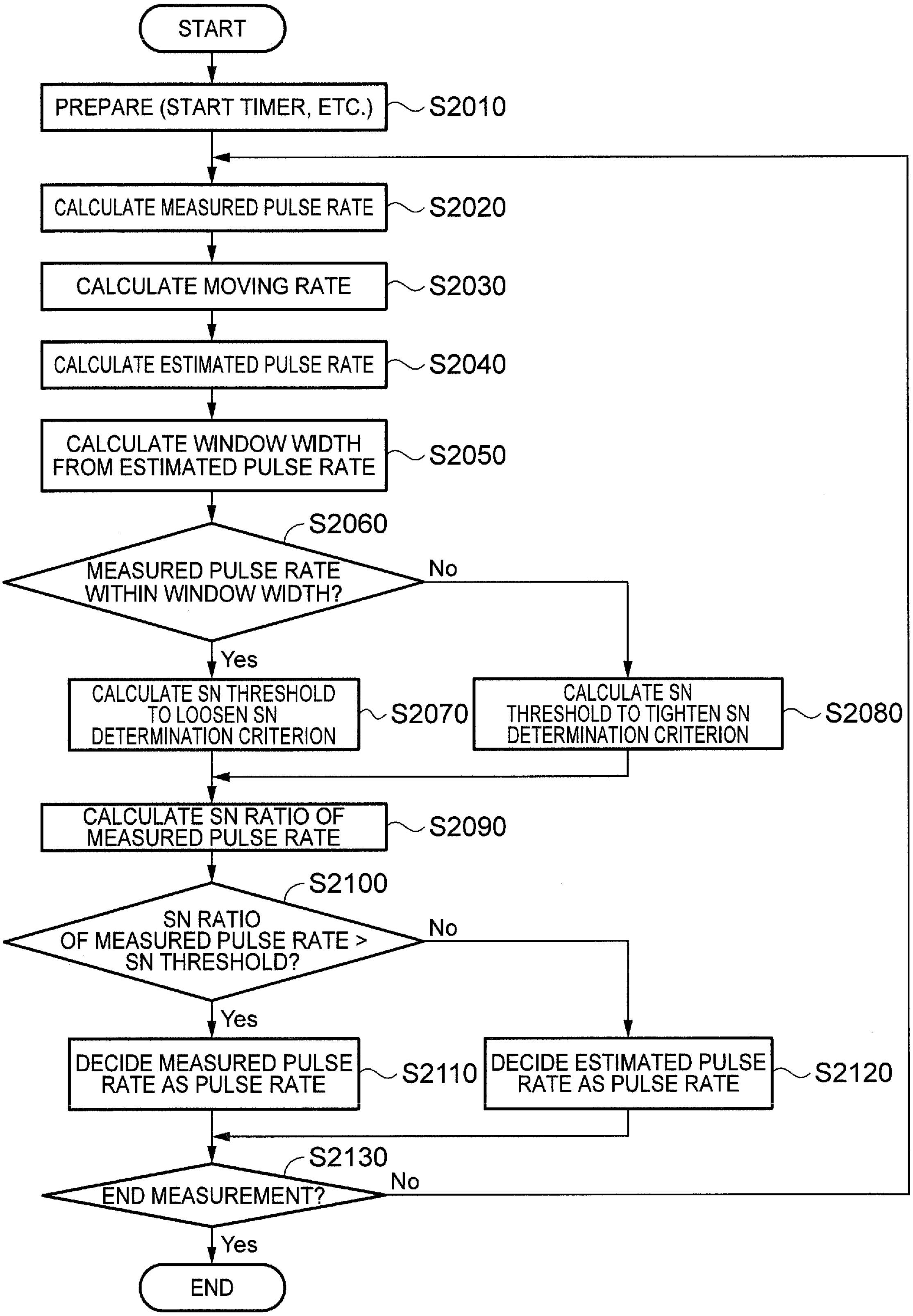
START
PREPARE (START TIMER, ETC.)
S2010
CALCULATE MEASURED PULSE RATE
S2020
CALCULATE MOVING RATE
S2030
CALCULATE ESTIMATED PULSE RATE
S2040
CALCULATE WINDOW WIDTH FROM ESTIMATED PULSE RATE
S2050
S2060
MEASURED PULSE RATE WITHIN WINDOW WIDTH?
No
Yes
CALCULATE SN THRESHOLD TO LOOSEN SN DETERMINATION CRITERION
S2070
CALCULATE SN THRESHOLD TO TIGHTEN SN DETERMINATION CRITERION
S2080
CALCULATE SN RATIO OF MEASURED PULSE RATE
S2090
S2100
SN RATIO OF MEASURED PULSE RATE > SN THRESHOLD?
No
Yes
DECIDE MEASURED PULSE RATE AS PULSE RATE
S2110
DECIDE ESTIMATED PULSE RATE AS PULSE RATE
S2120
S2130
END MEASUREMENT?
No
Yes
END

FIG. 16

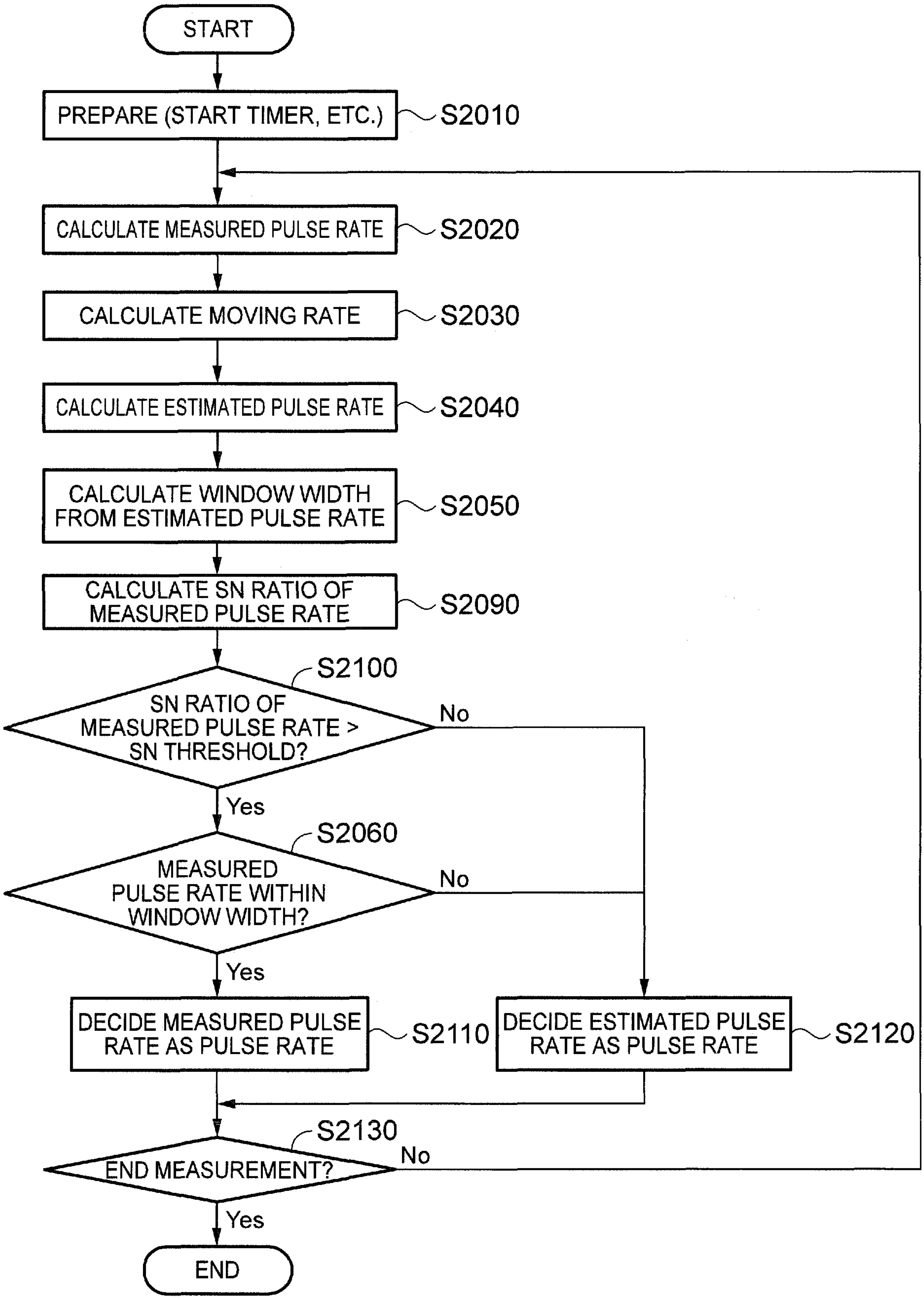
START
PREPARE (START TIMER, ETC.)
S2010
CALCULATE MEASURED PULSE RATE
S2020
CALCULATE MOVING RATE
S2030
CALCULATE ESTIMATED PULSE RATE
S2040
CALCULATE WINDOW WIDTH FROM ESTIMATED PULSE RATE
S2050
CALCULATE SN RATIO OF MEASURED PULSE RATE
S2090
S2100
SN RATIO OF MEASURED PULSE RATE > SN THRESHOLD?
No
Yes
S2060
MEASURED PULSE RATE WITHIN WINDOW WIDTH?
No
Yes
DECIDE MEASURED PULSE RATE AS PULSE RATE
S2110
DECIDE ESTIMATED PULSE RATE AS PULSE RATE
S2120
S2130
END MEASUREMENT?
No
Yes
END

FIG. 17

## INTERNATIONAL SEARCH REPORT

International application No. PCT/JP2014/002180

A. CLASSIFICATION OF SUBJECT MATTER
*A61B5/0245*(2006.01)i

According to International Patent Classification (IPC) or to both national classification and IPC

B. FIELDS SEARCHED

Minimum documentation searched (classification system followed by classification symbols)
A61B5/0245

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2014 |
| Kokai Jitsuyo Shinan Koho | 1971-2014 | Toroku Jitsuyo Shinan Koho | 1994-2014 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>A | JP 2012-232010 A (Seiko Epson Corp.), 29 November 2012 (29.11.2012), entire text; all drawings & US 2012/0283525 A1 & EP 2520222 A1 | 9,10,15<br>1-8,11-14, 16-20 |
| A | JP 2013-13644 A (Seiko Epson Corp.), 24 January 2013 (24.01.2013), entire text; all drawings & US 2013/0006123 A1 | 1-20 |
| A | JP 2013-13645 A (Seiko Epson Corp.), 24 January 2013 (24.01.2013), entire text; all drawings & US 2013/0006123 A1 | 1-20 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 08 May, 2014 (08.05.14) | 20 May, 2014 (20.05.14) |
| Name and mailing address of the ISA/<br>Japanese Patent Office<br>Facsimile No. | Authorized officer<br><br>Telephone No. |

Form PCT/ISA/210 (second sheet) (July 2009)

**INTERNATIONAL SEARCH REPORT**

International application No.
PCT/JP2014/002180

C (Continuation). DOCUMENTS CONSIDERED TO BE RELEVANT

| Category* | Citation of document, with indication, where appropriate, of the relevant passages | Relevant to claim No. |
|---|---|---|
| A | JP 2013-22246 A (Seiko Epson Corp.), 04 February 2013 (04.02.2013), entire text; all drawings (Family: none) | 1-20 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2012232010 A
- JP 2004081745 A
- JP 2013013644 A